# EUROPÄISCHE PATENTSCHRIFT

(11) **EP 4 415 790 B1**
(45) Veröffentlichungstag und Bekanntmachung des Hinweises auf die Patenterteilung: **09.07.2025**
(21) Anmeldenummer: 22830469.7
(22) Anmeldetag: 05.12.2022
(51) Int. Cl.: A61M 5/31, A61K 9/00, A61P 13/10

(54) **SPRITZE MIT INTEGRIERTEM ADAPTER**
SYRINGE WITH BUILT-IN ADAPTER
SERINGUE AVEC ADAPTATEUR INTÉGRÉ

(30) Priorität: 10.02.2022 DE 102022103164; 30.08.2022 DE 102022121864
(43) Veröffentlichungstag der Anmeldung: 21.08.2024
(73) Patentinhaber: Farco-Pharma GmbH, 50670 Köln (DE)
(72) Erfinder: GRUNOW, Markus, 50670 Köln (DE)
(74) Vertreter: Strehlke, Ingo Kurt
(86) Internationale Anmeldenummer: PCT/EP2022/084405
(87) Internationale Veröffentlichungsnummer: WO 2023/151847

(56) Entgegenhaltungen:
- EP-A1- 3 888 630
- EP-A1- 3 909 570
- EP-A2- 0 919 206
- EP-B1- 2 919 846
- WO-A1-2012/144026
- WO-A1-2017/069521
- DE-U1- 9 311 907
- JP-A- 2009 213 632
- US-A1- 2010 025 342
- US-A1- 2016 184 528
- US-A1- 2019 030 217

## Beschreibung

Die vorliegende Erfindung betrifft das medizinisch-technische Gebiet der medizinischen Instrumente in Form von medizinischen Spritzen, insbesondere Kolbenspritzen, wie sie zur Verabreichung von insbesondere medizinischen Zusammensetzungen eingesetzt werden, und zwar auch im Hinblick auf eine Applikation einer Zusammensetzung mittels Instillation in die Harnblase.

Die vorliegende Erfindung betrifft insbesondere eine Kolbenspritze, insbesondere Einweg-Kolbenspritze, insbesondere für die Applikation mittels Instillation einer bevorzugt oxybutyninhaltigen Zusammensetzung zur Verwendung bei der prophylaktischen bzw. therapeutischen Behandlung von neurogenen Blasenfunktionsstörungen, wobei die Kolbenspritze mit der oxybutyninhaltigen Zusammensetzung befüllt ist.

Weiterhin betrifft die vorliegende Erfindung auch ein entsprechendes Kit auf Basis der erfindungsgemäßen Kolbenspritze bzw. der oxybutyninhaltigen Zusammensetzung nach der Erfindung.

Darüber hinaus betrifft die vorliegende Erfindung auch die Verwendung einer Kolbenspritze in einem Verfahren zur Herstellung einer sterilen oxybutyninhaltigen Zusammensetzung sowie weiterhin auch die Verwendung einer Dampfsterilisation in einem Verfahren zur Herstellung einer sterilen oxybutyninhaltigen Zusammensetzung in einer vorzugsweise anwendungsfertigen Kolbenspritze.

Schließlich betrifft die vorliegende Erfindung auch die Verwendung einer Dampfsterilisation, insbesondere Wasserdampfsterilisation, in einem Verfahren zur Herstellung einer sterilen oxybutyninhaltigen Zusammensetzung in einer vorzugsweise anwendungsfertigen Kolbenspritze.

Der Wirkstoff Oxybutynin (synonym auch als 4-Diethylaminobut-2-inyl-2-cyclohexyl-2-hydroxy-2-phenylethanoat bezeichnet) bzw. Oxybutyninhydrochlorid ist ein Anticholinergikum, welches zur Behandlung von Blasenfunktionsstörungen bzw. Dysfunktionen der Harnblase eingesetzt wird, und zwar insbesondere von solchen Blasenfunktionsstörungen, welche mit einer Detrusorhyperaktivität und/oder eine Detrusor-Sphinkter-Dyssynergie einhergehen bzw. hierdurch bedingt sind.

Neurogene Blasenfunktionsstörungen (nBFS) bzw. neurogene Blasenentleerungsstörungen stellen eine oftmals therapiebedürftige pathologische Situation der Harnblase und der an der Harnspeicherung und Harnausscheidung beteiligten anatomischen Strukturen dar. Bei neurogenen Blasenfunktionsstörungen handelt es sich insbesondere um solche Dysfunktionen der Harnblase bzw. der relevanten anatomischen Strukturen, die infolge einer Fehlfunktion oder Verletzung des Nervensystems auftreten, beispielsweise infolge von Rückenmarksverletzungen, Spina bifida ("offener Rücken"), Diabetes, Multiple Sklerose, Schlaganfall oder Morbus Parkinson. Der mit der Dysfunktion der Harnblase einhergehende Leidensdruck von Patienten ist dabei mitunter sehr groß, zumal die oftmals bereits schon eingeschränkte Lebensqualität durch die mit der Dysfunktion einhergehenden Beschwerden zusätzlich verschlechtert wird.

Vor diesem Hintergrund besteht somit im Stand der Technik ein großer Bedarf an der Bereitstellung therapeutischer Konzepte, welche bei Vorliegen einer neurogenen Blasenfunktionsstörung zu einer Normalisierung bzw. Verbesserung der Blasenfunktion bzw. zu einer Verringerung der Symptome führen, und zwar sowohl was die Probleme beim Harnlassen als auch die vorliegenden hohen Drücke in der Harnblase anbelangt. Daher besteht ein wesentliches Ziel neben der Verbesserung der Miktion auch in der Verringerung des intrinsischen Drucks, um hierdurch etwaige Nierenschäden oder dergleichen zu vermeiden.

Vor diesem Hintergrund sind im Stand der Technik pharmakologische Therapien bekannt, bei denen auch Anticholinergika zum Einsatz kommen. In diesem Zusammenhang wird insbesondere auf die Verabreichung von Oxybutynin bzw. Oxybutyninhydrochlorid oder dergleichen abgestellt. Dabei besteht im Stand der Technik ein Fokus auf der systemischen bzw. oralen Applikation bzw. Verabreichung dieser Wirksubstanzen. Die systemische Verabreichung kann jedoch in verstärktem Maße mit systemischen Nebenwirkungen einhergehen. Darüber hinaus ist die Konzentration der Wirksubstanz am Wirkort, nämlich der Harnblase, mitunter nicht optimal einzustellen.

Überdies ist eine systemische Verabreichung der Wirksubstanz in Form von Anticholinergika insofern nachteilig, als ein Teil der verabreichten Wirksubstanz bei deren erster Passage im Gastrointestinalbereich bzw. in der Leber verstoffwechselt wird, wobei mitunter nicht wirksame Metaboliten entstehen, so dass die Bioverfügbarkeit am Wirkort geringer ausfällt bzw. die Wirkmenge am Wirkort nur schwer einzustellen bzw. vorzugeben ist. Folglich können Patienten mit systemischen Darreichungsformen mitunter nicht hinreichend eingestellt werden.

Darüber hinaus können Anticholinergika topisch appliziert werden, beispielsweise mittels eines transdermalen Pflasters oder dergleichen. Auch bei dieser systemischen Applikation können in erhöhtem Maße systemische Nebenwirkungen auftreten. Zudem besteht auch hier das Problem einer vorzeitigen Verstoffwechselung.

Darüber hinaus können Anticholinergika, wie Oxybutynin bzw. Oxybutyninhydrochlorid, topisch in den Urogenitalbereich, insbesondere in die Harnblase, appliziert bzw. instilliert werden, beispielsweise über einen Katheter oder dergleichen. Durch die diesbezüglich lokale Applikation bzw. die diesbezüglich vorgesehene Instillation erfolgt eine Darreichung der Wirksubstanz unmittelbar an seinem Wirkort, wodurch die gezielte Wirkung bzw. Bioverfügbarkeit erhöht und systemische Nebenwirkungen reduziert werden. Zudem wird der Wirkstoff bei dieser Verabreichungsart nicht vorzeitig über andere Organe verstoffwechselt. Überdies kann eine diesbezügliche Verabreichung gegebenenfalls auch von dem Patienten selbst vorgenommen werden.

Im Rahmen der im Stand der Technik vorgesehenen Instillationen bzw. topischen Applikationen in die Harnblase wird Oxybutynin bzw. Oxybutyninhydrochlorid im Allgemeinen in Form einer wässrigen Zusammensetzung bzw. wässrigen Lösung eingesetzt, wobei die wirkstoffhaltige Zusammensetzung bzw. Lösung unter Einsatz entsprechender Applikationsvorrichtungen in die Harnblase appliziert bzw. verabreicht wird.

Dabei kommt der diesbezüglichen Sterilität einer für die Instillation bzw. topischen Verabreichung in die Harnblase eingesetzte Zusammensetzung eine hohe Bedeutung zu, und zwar insbesondere vor dem Hintergrund, etwaige Infektionen der hierfür besonders anfälligen und mitunter durch das Krankheitsbild bereits vorgeschädigten Harnblase zu vermeiden.

Um eine Verkeimung zu vermeiden, können die wirkstoffhaltigen Zusammensetzungen beispielsweise unmittelbar vor deren Anwendung bzw. Verabreichung hergestellt werden, was aber aufwendig ist und zudem noch mit einer gewissen Gefahr der Kontamination der Zusammensetzung bei deren Herstellung einhergeht. Weiterhin können die wirkstoffhaltigen Zusammensetzungen als solche sterilisiert werden, beispielsweise mittels Mikrofiltrationsverfahren oder dergleichen, und anschließend in eine Aufbewahrungs- bzw. Applikationsvorrichtung abgefüllt werden. Auch hierbei kann es jedoch zu einer unerwünschten Kontamination kommen, insbesondere im Hinblick auf mögliche an der Aufbewahrungs- bzw. Verabreichungsvorrichtung anhaftende Keime oder dergleichen. **In** diesem Zusammenhang kann auch eine separate Sterilisation der Aufbewahrungs- bzw. Applikationsvorrichtung vorgesehen sein, was aber gleichermaßen zu mitunter nicht zufriedenstellenden Sterilisationsergebnissen führt, insbesondere bedingt durch das nachträgliche Abfüllen der Zusammensetzung in die Aufnahme- bzw. Applikationsvorrichtung. In diesem Zusammenhang kann zudem unter sterilen Bedingungen, beispielsweise unter Reinraumbedingungen, gearbeitet werden, was aber mit einem gewissen Aufwand einhergeht und zudem kostenintensiv ist.

Bei der Instillation bzw. topischen Applikation von wässrigen Zusammensetzungen mit Oxybutynin bzw. Oxybutyninhydrochlorid als Wirkstoff werden im Stand der Technik entsprechende Instillationsvorrichtungen auf Basis von Instillationsschläuchen bzw. (Harnblasen-)Kathetern eingesetzt, wobei die zu applizierende Zusammensetzung in einer Kolbenspritze eingebracht vorliegt und nach Einführen des Installationsschlauchs in die Harnblase durch manuelle Betätigung an der Kolbenspritze über den Instillationsschlauch bzw. Katheter in die Harnblase instilliert bzw. eingebracht wird. Zur Verbindung der Applikationsvorrichtung in Form der Kolbenspritze werden im Stand der Technik sogenannte Luer-Systeme und insbesondere Luer-Lock-Systeme verwendet, wobei ein separater Adapter, welcher eine Verbindung zwischen Kolbenspritze und Instillationsvorrichtung bzw. Instillationsschlauch ermöglicht, im Bereich mit der Austragsöffnung der Kolbenspritze über einen speziell ausgebildeten Anschluss lösbar verbunden wird. Die Instillationsvorrichtung bzw. der Instillationsschlauch wird anschließend mit seinem freien Ende mit dem Adapter und somit mit der Kolbenspritze verbunden bzw. an der Kolbenspritze angeschlossen.

Zwar ermöglichen die vorgenannten Systeme eine einfache Montage bzw. Ausbildung einer entsprechend lösbaren bzw. nicht dauerhaften Verbindung, da es sich bei dem Luer-System um eine Steckverbindung bzw. bei dem Luer-Lock-System um eine Verbindung handelt, bei welcher die Teile durch entsprechende Verdrehung weiterführend miteinander verbunden bzw. gesichert werden.

Luer- bzw. Luer-Lock-Systeme sind jedoch mit dem Nachteil verbunden, dass im Bereich der Verbindung zwischen Kolbenspritze einerseits und aufgesetztem Verbindungselement bzw. Adapter andererseits die Gefahr einer bakteriellen Kontamination besteht.

Zudem kann auch jedes bewusste oder unbewusste Lösen der Verbindung zwischen Kolbenspritze einerseits und Adapter andererseits das Eindringen von Bakterien bzw. Krankheitserregern ermöglichen, so dass auch von daher die Anzahl von Handhabungsschritten auf ein Minimum zu begrenzen ist. Zudem sollte zur Vermeidung von Kontaminationen unter aseptischen Bedingungen gearbeitet werden, einhergehend mit entsprechenden Desinfektionsmaßnahmen, beispielsweise was die Hände und das eingesetzte Material anbelangt, was aber wiederum mit einem gewissen Aufwand verbunden ist.

In diesem Zusammenhang ist auch problematisch, dass die lösbaren Verbindungen zwischen Spritze und Adapter nicht immer unter sämtlichen Umständen bzw. Anforderungen sicher schließen. So kann beispielsweise bei übermäßiger mechanischer Krafteinwirkung ein unbeabsichtigtes Lösen der Verbindung resultieren. Auch im Fall von schlecht gefertigten Verbindungselementen kann es zum spontanen Öffnen bzw. zum Lösen der Verbindung kommen, was mit einem unerwünschten Verlust an zu verabreichender Zusammensetzung einhergehen kann. Zudem kann die Verbindung insbesondere unbeabsichtigt sozusagen durch beliebige Personen gelöst werden, was im Sinne der Anwendungssicherheit gleichermaßen nachteilig sein kann.

Die zuvor geschilderten Unzulänglichkeiten der aus dem Stand der Technik bekannten Systeme wiegen umso schwerer, als dass Patienten, die intravesikales Oxybutynin anwenden, oft eine eingeschränkte Handmotorik aufweisen. So ist das Aufbringen eines Adapters auf eine Standardspritze für einige Patienten eine Herausforderung.

Hierzu ist auch beachtlich, dass die intravesikale Instillation oftmals im Rahmen des "intermittierenden Katheterismus" erfolgt. Unterschieden wird dabei zwischen dem intermittierenden Selbstkatheterismus (ISK) einerseits und dem intermittierenden Fremdkatheterismus (IFK) andererseits. Während beim ISK der oder die Betroffene sich selbst katheterisiert, erfolgt im Rahmen des IFK die Katheterisierung von einer anderen Person. Gemeinsam im Rahmen des ISK/IFK ist, dass die Durchführung unter Wahrung "aseptischer" Gesichtspunkte zu erfolgen hat, um Infektionen oder Kontaminationen vorzubeugen. Insbesondere ist dabei darauf zu achten, dass die Instillation über den Auslaufkanal durchgeführt wird und sicher und dicht am Harnauslauf bzw. Harnbeutelanschluss angesetzt wird.

Beachtlich ist in diesem Zusammenhang, dass im Rahmen des ISK/IFK eine Vielzahl von Kathetern, insbesondere Einmalkathetern, eingesetzt werden können. Dementsprechend liegt für die in Rede stehende Anwendung eine hohe bzw. prinzipiell unbeschränkte Vielzahl an Kathetern, insbesondere Einmalkathetern, vor, die im Rahmen der intravesikalen Verabreichung einsetzbar sind.

Die EP 3 888 630 A1 betrifft ein Verfahren zur Herstellung einer sterilen oxybutyninhaltigen Zusammensetzung in einer vorzugsweise anwendungsfertigen Kolbenspritze, wobei die Kolbenspritze einen speziellen Spritzenkörper bzw. Spritzenzylinder und einen Spritzenkolben mit einem speziellen Kolbenstopfen aufweist. Bei dem Verfahren soll die Kolbenspritze mit der oxybutyninhaltigen Zusammensetzung befüllt werden, wonach anschließend eine Dampfsterilisation durchgeführt wird.

Weiterhin betrifft die US 2016/184528 A1 einen Spritzenzylinder mit einem hohlen Zylinderabschnitt, der an einem proximalen Ende des Zylinderabschnitts eine Öffnung aufweist und so konfiguriert ist, dass eine Dichtung von der Öffnung am proximalen Ende in den Zylinderabschnitt eingeführt werden kann. Ein innerer Umfangsabschnitt des Zylinderabschnitts umfasst einen sich verjüngenden Abschnitt in einem Hauptbereich des inneren Umfangsabschnitts in einer axialen Richtung, wobei der sich verjüngenden Abschnitt einen speziell ausgebildeten Innendurchmesser aufweisen soll.

Die US 2010/025342 A1 betrifft einen Separator für plättchenreiches Plasma, welcher eine erste Spritze zum Absaugen von Blut und eine zweite Spritze zum Absaugen eines zentrifugierten Abschnitts, welcher Blutplättchen und Plasma enthält, aus der ersten Spritze, umfasst.

Die EP 2 919 846 A2 betrifft einen Katheter bzw. ein Kit, welches zur Behandlung der Prostata bzw. der Harnblase eingesetzt wird. Diesbezüglich soll ein Katheter in Form eines Verweilkatheters eingesetzt werden, wobei der Katheter einen Katheterschlauch, einen am Katheterschlauch befindlichen Katheteranschluss und einen befüllbaren bzw. expandierbaren Prostataballon aufweist. Zudem soll eine mindestens ein Medikament enthaltende Applikationsvorrichtung insbesondere in Form einer Spritze bzw. Kolbenspritze zur Verabreichung eines Medikaments eingesetzt werden.

Die EP 0 919 206 A2 betrifft eine Kartusche, bestehend aus einem Körperabschnitt mit einem offenen Ende und einem Ende mit verringertem Durchmesser, einen Halteabschnitt neben dem offenen Ende des Körperabschnitts, einer zerbrechlichen Dichtung, die einem Durchgang zugeordnet ist, welcher im Endbereich mit verringertem Durchmesser ausgebildet ist, und einen Abgabeabschnitt, der an dem Ende mit verringertem Durchmesser des Körperabschnitts angeordnet werden kann.

Die US 2019/030217 A1 betrifft ein flüssiges Material zur Beschichtung medizinischer Geräte auf, wobei das Material spezielle Polysiloxane enthalten soll. Diesbezüglich wird beispielsweise auf die Beschichtung von Kolbenstopfen abgestellt, welche eine gleitfähige Beschichtungsschicht aufweisen sollen.

Die DE 93 11 907 U1 betrifft eine Spritze für medizinische Zwecke mit einem zylindrischen Körper, einem in dem Körper gradlinig verschiebbaren Kolben, einem ebenfalls in dem Körper und in Bezug auf den Kolben geradlinig verschiebbaren Nadelhalter mit einer Nadel, wobei die Nadel sich vor Ingebrauchnahme hermetisch geschützt im Inneren des Zylinders befindet und wobei die Nadel von dem Nadelhalter gehalten wird. Zudem soll der Körper der Spritze an seinem distalen Ende von einem von der Nadel durchstechbaren deckelartigen Gebilde aus Elastomer verschlossen sein, wobei der Nadelhalter und das distale Ende des Körpers einrastbare Kupplungselemente aufweisen und wobei das proximale Ende des Nadelhalters und der Kolben einrastbar nach dem Einrasten nicht wieder ausrastbare Kupplungselemente aufweisen. Zudem soll ein bestimmter Mindestabstand zwischen dem Kolben und dem Nadelhalter von einem Teil bestimmt sein, dessen Widerstand gegen Zusammendrücken im feuchten oder nassen Zustand geringer ist als im trockenen Zustand.

Die WO 2017/069521 A1 betrifft einen Sicherheitsfilter bzw. eine Spritze, welche einen derartigen Sicherheitsfilter aufweist. Der Sicherheitsfilter soll einen ersten Körper und einen zweiten Körper sowie eine Filterbewegungseinheit, welche verschiebbar zwischen dem ersten Körper und dem zweiten Körper montiert ist, aufweisen.

Die JP 2009 213632 A betrifft eine Spritze, bei welcher der Zylinderkopf mit einer Verbindungsöffnung auf Basis einer sogenannten Luer-Verbindung ausgerüstet ist.

Weiterhin betrifft die WO 2012/144026 A1 einen (Spritzen-)Zylinder mit angebrachter Kappe bzw. diesbezügliche Spritzen, wobei die Verbindungsanschlüsse als Luer-Lok-Typ sowie als Slip-in-Typ ausgebildet sein sollen.

Die EP 3 909 570 A1 betrifft eine oxybutyninhaltige Zusammensetzung zur Verwendung bei der prophylaktischen bzw. therapeutischen Behandlung von insbesondere neurogenen Blasenfunktionsstörungen.

Da es im Stand der Technik keine einheitliche Normenauswahl für Urinbeutelanschlüsse gibt, können somit erhebliche Abweichungen der untersuchten Katheter in Material und Durchmesser des Urinbeutelanschlusses festgestellt werden. Neben unterschiedlichen Durchmessern sind jedoch auch unterschiedliche Materialien wie Latex, Silikon, PVC und Nelaton festzustellen, die wiederum erheblichen Einfluss auf die Haftfähigkeit und Dichtigkeit des Anschlusses aufweisen. So weist Silikon eine deutlich bessere Haft- und Dehnfähigkeit auf, wohingegen andere Materialien diesbezüglich nachteilige Eigenschaften aufweisen, insbesondere unter Einsatzbedingungen, beispielsweise aufgrund eines befeuchteten Katheters.

Zusammenfassend lässt sich somit feststellen, dass die aus dem Stand der Technik bekannten Spritzen-Konzepte, insbesondere im Zusammenhang mit der Verabreichung einer Zusammensetzung und/oder zum Anschluss an eine Instillationsvorrichtung, unzureichend bzw. verbesserungsbedürftig sind, insbesondere in Bezug auf eine einfache Handhabung mit möglichst geringem Kraftaufwand im Rahmen der Katheterverbindung sowie auf eine Kompatibilität mit möglichst verschiedenen Katheter-Konzepten bei gleichzeitig sicherer Abdichtung.

Vor diesem Hintergrund besteht eine Aufgabe der vorliegenden Erfindung somit darin, eine insgesamt effiziente und vorzugsweise anwendungsfertige Aufbewahrungs- bzw. Applikationsvorrichtung bereitzustellen, nämlich eine Kolbenspritze, insbesondere für die Verabreichung bzw. Instillation einer Oxybutynin bzw. Oxybutyninhydrochlorid als Wirkstoff enthaltenen Zusammensetzung in die Harnblase bzw. Blase, bereitzustellen, wobei die zuvor geschilderten Nachteile des Standes der Technik zumindest weitgehend vermieden oder aber wenigstens abgeschwächt werden sollen.

Insbesondere ist es eine Aufgabe der vorliegenden Erfindung, eine diesbezügliche Aufbewahrungs- bzw. Applikationsvorrichtung bereitzustellen, welche eine einfache Handhabung mit möglichst geringem Kraftaufwand beim Verbinden mit einem zugeordneten Katheter sicherstellt sowie eine hohe Kompatibilität mit verschiedensten Blasenkathetern bei einer sicheren und dichten Verbindung gewährleistet.

Vorliegend ist eine Aufgabe der vorliegenden Erfindung auch darin zu sehen, ein entsprechendes Konzept für die Verabreichung einer Oxybutynin bzw. Oxybutyninhydrochlorid als Wirkstoff enthaltenen Zusammensetzung zur Instillation in die Harnblase bzw. Blase bereitzustellen, welches bei einfacher und schneller Handhabung zudem eine hohe Anwendungssicherheit gewährleistet. Zudem sollen die entsprechenden Prozesse bzw. Schritte zur Bereitstellung einer solchen Aufbewahrungs- bzw. Applikationsvorrichtung vereinfacht werden, wobei zudem der Einsatz von Verpackungsmaterial reduziert werden soll.

Vor diesem Hintergrund besteht eine weitere Aufgabe der vorliegenden Erfindung auch darin, ein weiterführendes Konzept für die Herstellung einer sterilen Zusammensetzung, welche Oxybutynin bzw. Oxybutyninhydrochlorid als Wirkstoff aufweist, und welche in einer vorzugsweise anwendungsfertigen Aufbewahrungs- bzw. Applikationsvorrichtung, nämlich einer Kolbenspritze, vorliegt, bereitzustellen, wobei gleichermaßen die zuvor geschilderten Nachteile des Standes der Technik zumindest weitgehend vermieden oder aber wenigstens abgeschwächt werden sollen.

Insbesondere soll im Rahmen einer nochmals weiteren Aufgabe der vorliegenden Erfindung auch eine mit einer wirkstoffhaltigen Zusammensetzung befüllte Aufbewahrungs- bzw. Applikationsvorrichtung, und zwar in Form einer (Einweg-) Spritze bzw. Kolbenspritze, bereitgestellt werden, welche in verbesserter Weise einem Sterilisationsverfahren zugänglich ist, wobei eine effektive Sterilisation unter Beibehaltung der Stabilität des Befüllungsguts in Form der wirkstoffhaltigen Zusammensetzung gewährleistet werden soll.

Zur Lösung der zuvor geschilderten Aufgabe schlägt die vorliegende Erfindung somit - gemäß einem **ersten** Aspekt der vorliegenden Erfindung - eine Kolbenspritze gemäß Patentanspruch 1 vor, insbesondere für die Applikation mittels Instillation einer bevorzugt sterilen oxybutyninhaltigen Zusammensetzung zur Verwendung bei der prophylaktischen bzw. therapeutischen Behandlung von neurogenen Blasenfunktionsstörungen, vor, wobei die Kolbenspritze mit der bevorzugt sterilen oxybutyninhaltigen Zusammensetzung befüllt ist. Jeweils vorteilhafte Weiterbildungen und Ausgestaltungen dieses Erfindungsaspekts sind Gegenstand der die mit der oxybutyninhaltigen Zusammensetzung befüllten Kolbenspritze betreffenden Unteransprüche.

Gegenstand der vorliegenden Erfindung ist weiterhin - gemäß einem **zweiten** Aspekt der vorliegenden Erfindung - auch das erfindungsgemäße Kit (*Kit-of-parts*), gemäß dem diesbezüglichen, das erfindungsgemäße Kit betreffenden unabhängigen Patentanspruch.

Nochmals weiterer Gegenstand der vorliegenden Erfindung - gemäß einem **dritten** Aspekt der vorliegenden Erfindung - ist auch die erfindungsgemäße Verwendung einer erfindungsgemäßen Kolbenspritze in einem Verfahren zur Herstellung einer sterilen oxybutyninhaltigen Zusammensetzung.

Ein weiterer Gegenstand der vorliegenden Erfindung - gemäß einem **vierten** Aspekt der vorliegenden Erfindung - ist auch die erfindungsgemäße Verwendung einer Dampfsterilisation, insbesondere Wasserdampfsterilisation, in einem Verfahren zur Herstellung einer sterilen oxybutyninhaltigen Zusammensetzung in einer vorzugsweise anwendungsfertigen Spritze, insbesondere in einer erfindungsgemäßen Kolbenspritze.

Es versteht sich von selbst, dass Ausgestaltungen, Ausführungsformen, Vorteile und dergleichen, welche nachfolgend zu Zwecken der Vermeidung von Wiederholungen zu einem Erfindungsaspekt aufgeführt sind, selbstverständlich auch in Bezug auf die übrigen Erfindungsaspekte entsprechend gelten, ohne dass dies einer gesonderten Erwähnung bedarf.

Darüber hinaus verhält es sich im Hinblick auf die nachfolgende Beschreibung der vorliegenden Erfindung auch derart, dass die jeweils im Zusammenhang mit den speziellen Ausgestaltungen, Ausführungsformen, Vorteilen, Beispielen oder dergleichen angeführten Merkmale der vorliegenden Erfindung auch in deren Kombination als offenbart gelten. Somit gelten vorliegend auch übergeordnete Kombinationen einzelner oder mehrerer Merkmale, welche für jeweilige Ausgestaltungen, Ausführungsformen, Anwendungsbeispiele oder dergleichen angeführt sind, als offenbart.

Bei allen nachstehend genannten relativen bzw. prozentualen gewichtsbezogenen Angaben, insbesondere Mengen- bzw. Konzentrationsangaben, ist weiterhin zu beachten, dass diese im Rahmen der vorliegenden Erfindung vom Fachmann derart auszuwählen sind, dass sie sich in der Summe unter Einbeziehung aller Komponenten bzw. Inhaltsstoffe, insbesondere wie nachfolgend definiert, zu 100 % bzw. 100 Gew.-% ergänzen bzw. addieren; dies versteht sich aber für den Fachmann von selbst.

Weiterhin gilt, dass der Fachmann - anwendungsbezogen oder einzelfallbedingt - von den nachfolgend angeführten Konzentrations-, Gewichts-, Mengen- und Bereichsangaben abweichen kann, ohne dass er den Rahmen der vorliegenden Erfindung verlässt.

Zudem gilt, dass alle im Folgenden genannten Werte- bzw. Parameterangaben oder dergleichen grundsätzlich mit genormten bzw. standardisierten oder explizit angegebenen Bestimmungsverfahren und andernfalls mit dem Fachmann auf diesem Gebiet an sich geläufigen Bestimmungs- bzw. Messmethoden ermittelt bzw. bestimmt werden können. Sofern nicht anders angegeben, werden die zugrundeliegenden Werte bzw. Parameter unter Standardbedingungen (d. h. insbesondere bei einer Temperatur von 20 °C und/oder bei einem Druck von 1.013,25 hPa bzw. 1,01325 bar) ermittelt.

Zu Zwecken der Veranschaulichung der vorliegenden Erfindung wird in der nachfolgenden Beschreibung der erfindungsgemäßen Gegenstände auch auf die in der Figur angeführten Bezugszeichen zurückgegriffen; die diesbezügliche Anführung der Bezugszeichen ist dabei rein veranschaulichend und geht mit keinerlei Beschränkung der erfindungsgemäßen Gegenstände einher.

Dies vorausgeschickt wird im Folgenden die vorliegende Erfindung im Detail erläutert:
Gegenstand der vorliegenden Erfindung - gemäß einem **ersten** Aspekt der vorliegenden Erfindung - ist eine Kolbenspritze, insbesondere Einweg-Kolbenspritze,
wobei die Kolbenspritze
   - einen Spritzenkörper, welcher einen Spritzenzylinder, zur Aufnahme einer vorzugsweise oxybutyninhaltigen Zusammensetzung, und einen Adapter zum Anschluss an eine Instillationsvorrichtung aufweist, wobei der Spritzenkörper einstückig ausgebildet ist und/oder wobei der Adapter mit dem Spritzenzylinder fest und/oder unlösbar verbunden, vorzugsweise stoffschlüssig verbunden, ist, und
   - einen Spritzenkolben mit einem Kolbenstopfen aufweist,
wobei der Adapter mindestens einen, vorzugsweise genau einen, ersten Abschnitt mit einem sich in Auslassrichtung kontinuierlich verjüngenden Außenumfang und/oder Außendurchmesser aufweist, und
wobei der Adapter mindestens einen, vorzugsweise genau einen, sich in Auslassrichtung unmittelbar an den ersten Abschnitt anschließenden zweiten, zylindrischen Abschnitt mit einem gegenüber dem ersten Abschnitt) verringerten Außenumfang aufweist,
wobei sich der zweite Abschnitt hin zu einer Auslassöffnung des Adapters erstreckt und die Auslassöffnung am freien Ende des zweiten Abschnitts ausgebildet ist,
wobei sich der erste Abschnitt (ausgehend von einem maximalen Außendurchmesser des ersten Abschnitts in Auslassrichtung (AR) zu einem minimalen Außendurchmesser des ersten Abschnitts verjüngt und das Verhältnis des maximalen Außendurchmessers des ersten Abschnitts zu dem minimalen Außendurchmesser des ersten Abschnitts in einem Bereich von 1,1 bis 1,6 liegt,
wobei am Übergang vom ersten Abschnitt zum zweiten Abschnitt genau eine Stufe ausgebildet ist,
wobei wenigstens ein dem ersten Abschnitt unmittelbar vorgelagerter dritter Abschnitt des Adapters vorgesehen ist,
wobei der dritte Abschnitt einen sich in Auslassrichtung kontinuierlich verjüngenden Außenumfang und/oder Außendurchmesser aufweist, wobei der dritte Abschnitt stufenfrei in den ersten Abschnitt übergeht und
wobei das Verhältnis eines maximalen Außendurchmessers des dritten Abschnitts zu einem minimalen Außendurchmesser des dritten Abschnitts (A₃) in einem Bereich von 1,01 bis 1,06 liegt.

Eine zentrale Idee der vorliegenden Erfindung ist zunächst darin zu sehen, dass erfindungsgemäß eine sehr spezielle Kolbenspritze bereitgestellt wird, wobei die Kolbenspritze einen einstückig (einteilig) ausgebildeten Spritzenkörper mit einem Spritzenzylinder und einem fest bzw. dauerhaft mit dem Spritzenzylinder verbundenen Adapter aufweist bzw. bei welcher der Adapter mit dem Spritzenzylinder, insbesondere unlösbar, verbunden ist.

Darauf aufbauend liegt die erfindungsgemäße Weiterentwicklung insbesondere darin, den Adapter mit mindestens einem, vorzugsweise genau einem, ersten Abschnitt mit einem sich in Auslassrichtung kontinuierlich verjüngenden und/oder verringernden Außendurchmesser auszurüsten. Dieser vorzugsweise genau eine erste Abschnitt bildet dabei vorzugsweise den Hauptabschnitt des erfindungsgemäßen Adapters und ermöglicht eine sichere und/oder komfortable Anbindung an zugeordnete Vorrichtungen, insbesondere an zugeordnete Katheter, wobei aufgrund der verjüngenden Ausbildung eine einfache, insbesondere selbstzentrierende, Anbindung bei hoher Dichtigkeit sichergestellt ist. Überdies wird aufgrund der verjüngenden Ausgestaltung eine hohe Kompatibilität an sämtliche, herkömmliche Blasenkatheter sichergestellt.

Wie seitens der Anmelderin in diesem Zusammenhang in vollkommen überraschender Weise herausgefunden wurde, lässt sich das erfindungsgemäße Ziel einer einfachen Anwendung, hohen Dichtigkeit sowie einem hohen Grad an Kompatibilität dadurch weiterführend steigern, indem mindestens ein, vorzugsweise genau ein, sich in Auslassrichtung unmittelbar an den ersten Abschnitt anschließenden zweiter, zylindrischer Abschnitt mit einem gegenüber dem ersten Abschnitt verringerten Außenumfang und/oder Außendurchmesser vorgesehen ist.

In diesem Zusammenhang bildet der auf den ersten Abschnitt folgende zweite Abschnitt vorzugsweise eine Ergänzung und/oder Verstärkung des ersten Abschnitts, insbesondere dahingehend, dass die Passgenauigkeit und/oder Dichtigkeit an anzuschließende Vorrichtungen und/oder Katheter weiterführend verstärkt wird.

Insofern kann der zweite Abschnitt - ohne sich auf eine bestimmte Theorie beschränken zu wollen - als Dichtigkeitsunterstützung im Zusammenhang mit dem ersten Abschnitt fungieren, wobei keine Beeinträchtigung im Hinblick auf die durch den ersten Abschnitt sichergestellte Kompatibilität vorliegt. Alternativ oder zusätzlich kann der zweite Abschnitt auch so angepasst werden, dass eine dichtende Anbindung an ein zugeordnetes Verschlusselement und/oder eine zugeordnete Dichtkappe ermöglicht wird. Besonders bevorzugt ist der zweite Abschnitt in diesem Zusammenhang zumindest teilweise an das zugeordnete Verschlusselement angepasst, insbesondere komplementär zur Ausbildung einer dichtenden Anlage zwischen Verschlusselement und dem zweiten Abschnitt.

In diesem Zusammenhang kann die geometrische Ausgestaltung des zweiten Abschnitts prinzipiell beliebig sein, um das durch die vorliegende Erfindung angestrebte Ziel einer hohen Kompatibilität bei hoher Dichtigkeit zu erfüllen. Erfindungsgemäß ist der zweite Abschnitt dabei zylindrisch ausgebildet, was besonders vorteilhaft in Bezug auf eine Anbindung einer standardmäßigen Dichtkappe bzw. eines sonstigen Verschlusselements ist. Vor diesem Hintergrund kann der zweite Abschnitt vorzugsweise standardmäßig und/oder analog zu einer Standardspritze ausgebildet sein.

Im Ergebnis kann somit aufgrund der erfindungsgemäßen Ausgestaltung des Adapters realisiert werden, dass herkömmlich ausgebildete Verschlusskappen eingesetzt werden können, einhergehend mit einer hohen Kompatibilität der erfindungsgemäßen Kolbenspritze an zugeordnete Applikationsvorrichtungen.

Unter dem Begriff "Auslassrichtung", wie er im Rahmen der vorliegenden Erfindung verwendet wird, ist vorzugsweise eine Austrittsrichtung bzw. Fließrichtung der Flüssigkeit insbesondere im Rahmen der Betätigung der Kolbenspritze zu sehen, die vorzugsweise zumindest im Wesentlichen der Axialrichtung bzw. Längserstreckung der Kolbenspritze, insbesondere des Adapters, entspricht.

Innerhalb des ersten Abschnitts erfolgt eine Verjüngung insbesondere stufenfrei und/oder unterbrechungsfrei, wodurch eine sichere Verbindungsfläche mit einem zugeordneten Katheter oder einer sonstigen Applikationsvorrichtung vorgesehen ist. Überdies wird durch den sich anschließenden zweiten Abschnitt, der zylindrisch ausgebildet ist und einen gegenüber dem ersten Abschnitt verringerten Außendurchmesser aufweist, sichergestellt, dass eine weitere Anbindung an eine Applikationsvorrichtung ermöglicht wird, einhergehend mit einer festen Verbindung sowie der Möglichkeit, herkömmliche Verschlusselemente bzw. Dichtkappen oder sonstige Adapter sicher und komfortabel an den Adapter anzubinden.

Eine besonders vorteilhafte Ausführung der erfindungsgemäßen Kolbenspritze bzw. des erfindungsgemäßen Konzepts liegt dabei, wie nachfolgend im Detail erläutert, darin, die Dimensionierung der Abschnitte des Adapters, insbesondere im Hinblick auf deren Durchmesser und/oder Längen, gezielt aufeinander abzustimmen. Dadurch werden entsprechende Vorteile im Hinblick auf eine hohe Dichtigkeit, einfache Handhabung sowie hohe Kompatibilität entsprechend weiterführend erzielt bzw. in besonders zweckgerichteter Weise erfüllt.

Dies vorausgeschickt wird die vorliegende Erfindung nunmehr nachfolgend im Detail auch anhand von bevorzugten Ausführungsformen bzw. Ausführungsbeispielen darstellenden Zeichnungen bzw. Figurendarstellungen beschrieben. Im Zusammenhang mit der Erläuterung dieser bevorzugten Ausführungsform bzw. Ausführungsbeispiele der vorliegenden Erfindung, welche jedoch in Bezug auf die vorliegende Erfindung keinesfalls beschränkend sind, werden auch weitergehende Vorteile, Eigenschaften, Aspekte und Merkmale der vorliegenden Erfindung aufgezeigt.

In den Figurendarstellungen zeigt:

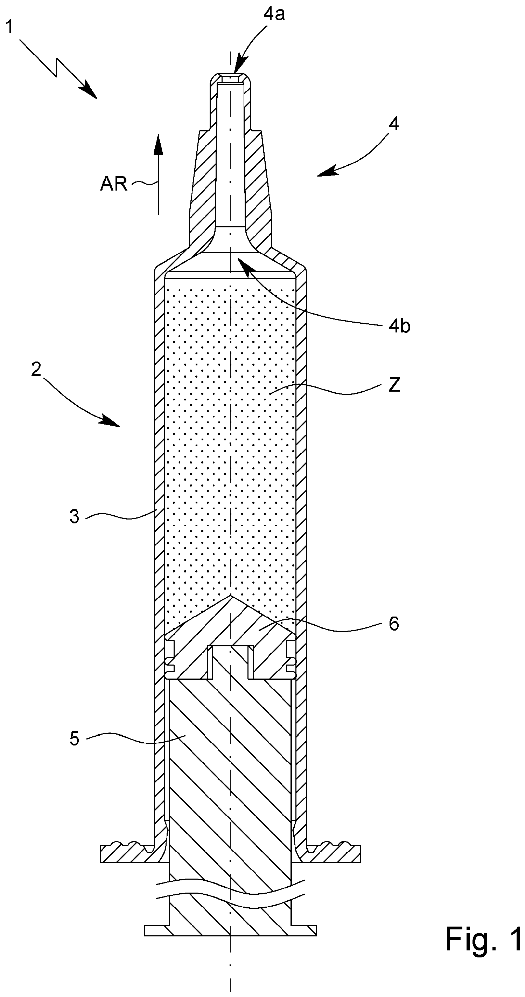

- Fig. 1: eine schematische Schnittdarstellung (Querschnitt entlang der Längsachse der Kolbenspritze) einer erfindungsgemäßen Kolbenspritze, welche mit einer bevorzugt sterilen oxybutyninhaltigen Zusammensetzung befüllt ist, wobei die Kolbenspritze nach der Erfindung einen Spritzenkolben mit einem Kolbenstopfen, einen Spritzenkörper mit einem Spritzenzylinder, insbesondere zur Aufnahme der oxybutyninhaltigen Zusammensetzung, und einen Adapter, insbesondere zum Anschluss einer Instillationsvorrichtung, aufweist; Fig. 1 verdeutlicht dabei, dass der Spritzenkörper einstückig bzw. einteilig ausgebildet ist bzw. dass der Adapter mit dem Spritzenzylinder fest bzw. unlösbar verbunden, vorzugsweise stoffschlüssig verbunden, ist; zudem zeigt Fig. 1 eine bevorzugte Ausführungsform der vorliegenden Erfindung, wonach der Adapter einen ersten, sich verjüngenden Abschnitt und einen sich in Auslassrichtung daran anschließenden zweiten, zylindrischen Abschnitt mit einem gegenüber dem ersten Abschnitt reduzierten Außendurchmesser aufweist;

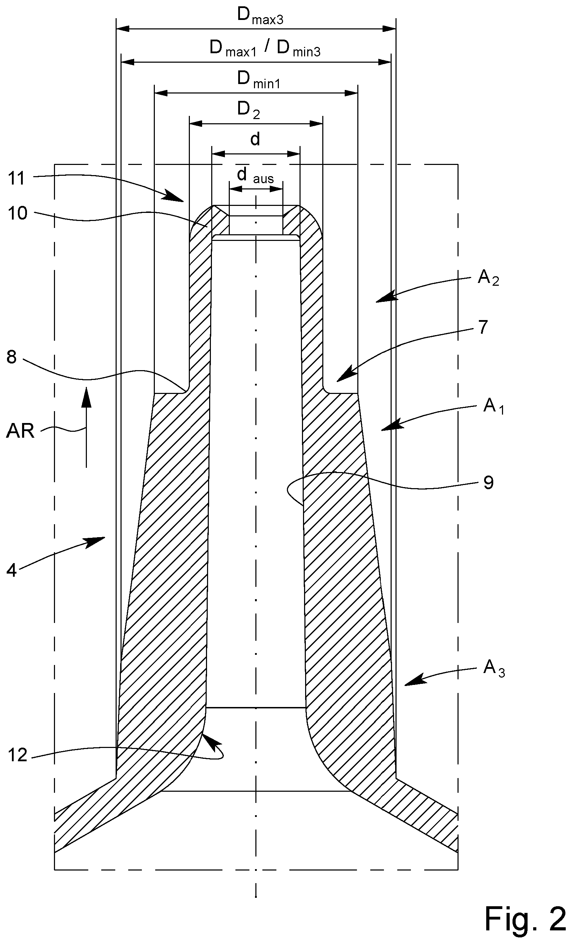

- Fig. 2: eine schematische, ausschnittsweise Vergrößerung der erfindungsgemäßen Kolbenspritze im Bereich des Adapters der erfindungsgemäßen Kolbenspritze; Fig. 2 veranschaulicht dabei die unterschiedlichen Außenumfänge bzw. Durchmesser der einzelnen, den Adapter der erfindungsgemäßen Kolbenspritze ausbildenden Abschnitte;

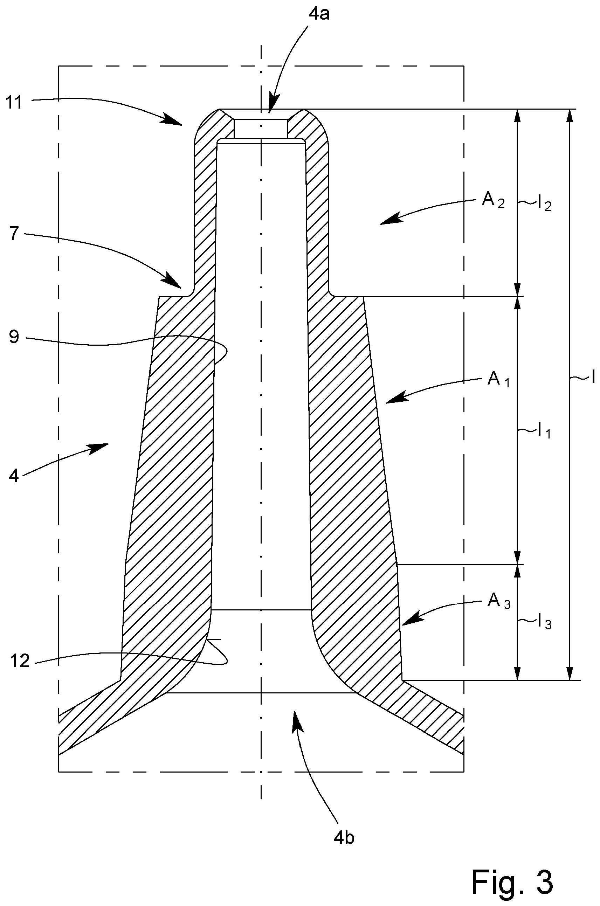

- Fig. 3: eine weitere vergrößerte Ansicht der erfindungsgemäßen Kolbenspritze im Bereich des Adapters; Fig. 3 veranschaulicht dabei die einzelnen Längenverhältnisse der einzelnen, den Adapter ausbildenden Abschnitte der erfindungsgemäßen Kolbenspritze;

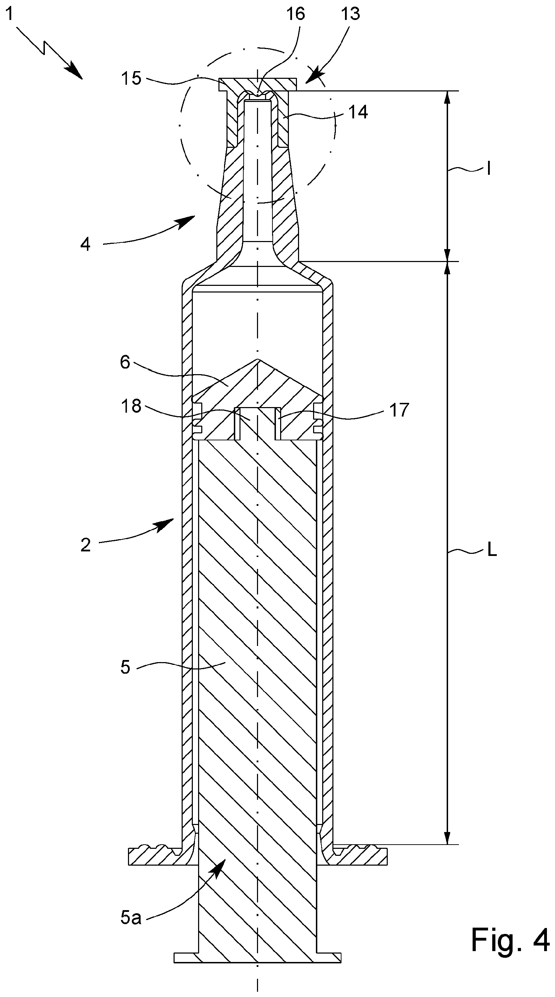

- Fig. 4: eine weitere schematische Schnittdarstellung (Querschnitt entlang der Längsachse der Kolbenspritze) der erfindungsgemäßen Kolbenspritze, wobei die Kolbenspritze nach der Erfindung einen Spritzenkolben mit einem Kolbenstopfen, einen Spritzenkörper mit einem Spritzenzylinder, insbesondere zur Aufnahme der oxybutyninhaltigen Zusammensetzung, und einen Adapter, insbesondere zum Anschluss einer Instillationsvorrichtung, aufweist; Fig. 4 verdeutlicht dabei insbesondere bzw. zudem, dass gemäß der in Fig. 4 dargestellten bevorzugten Ausführungsform der vorliegenden Erfindung der Adapter mitsamt der Auslassöffnung des Adapters mit einem speziellen Verschlusselement in Form einer Verschlusskappe verschlossen bzw. bedeckt ist;

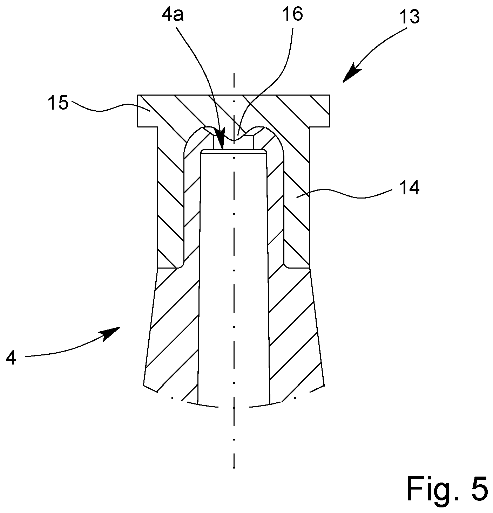

- Fig. 5: eine ausschnittsweise Vergrößerung der in Fig. 4 gezeigten Darstellung, und zwar im Bereich des freien Endes des Adapters, der mit dem erfindungsgemäß vorgesehenen Verschlusselement in der gezeigten bevorzugten Ausführungsform verschlossen ist; Fig. 5 illustriert dabei den exakten und bevorzugten Aufbau des erfindungsgemäß vorgesehenen Verschlusselements zum vorzugsweise dichtenden Verschließen des freien Endes des Adapters bzw. der Auslassöffnung des Adapters; und

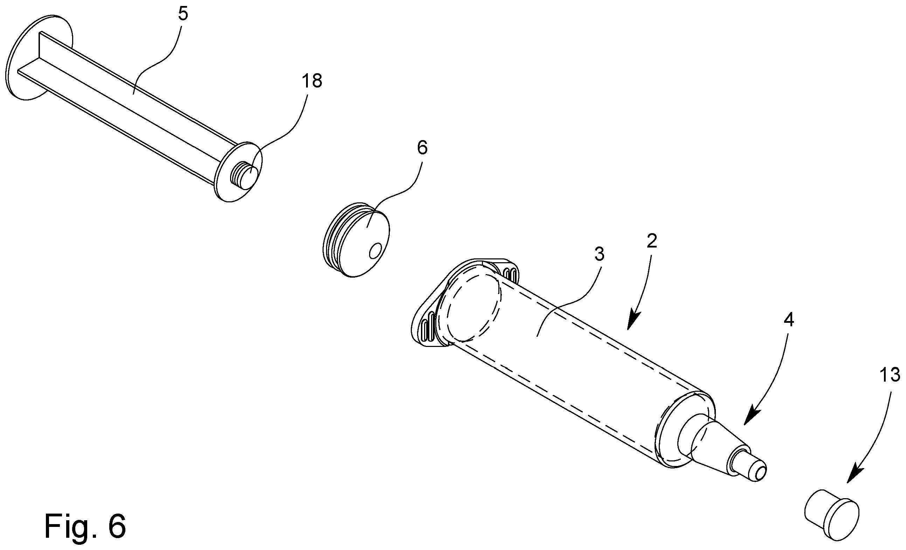

- Fig. 6: eine schematische Darstellung (Explosionsdarstellung) der Kolbenspritze nach der Erfindung mit den der Kolbenspritze zugrundeliegenden Komponenten bzw. Bestandteilen in einer perspektivischen Draufsicht; Fig. 6 zeigt dabei den Spritzenzylinder sowie den Adapter aufweisenden und einstückig ausgebildeten Spritzenkörper sowie den Spritzenkolben mit angebrachtem Kolbenstopfen und weiterhin das Verschlusselement bzw. die Verschlusskappe.

In diesem Zusammenhang betrifft die vorliegende Erfindung gemäß dem vorliegenden Aspekt somit eine insbesondere auf Basis der Figurendarstellungen beschriebene Kolbenspritze 1, insbesondere Einweg-Kolbenspritze,
wobei die Kolbenspritze 1
   - einen Spritzenkörper 2, welcher einen Spritzenzylinder 3, insbesondere zur Aufnahme einer vorzugsweise oxybutyninhaltigen Zusammensetzung Z, und einen Adapter 4 zum Anschluss an eine Instillationsvorrichtung aufweist, wobei der Spritzenkörper 2 einstückig ausgebildet ist und/oder wobei der Adapter 4 mit dem Spritzenzylinder 3 fest und/oder unlösbar verbunden, vorzugsweise stoffschlüssig verbunden, ist, und
   - einen Spritzenkolben 5 mit einem Kolbenstopfen 6
   aufweist,
wobei der Adapter 4 mindestens einen, vorzugsweise genau einen, ersten Abschnitt A₁ mit einem sich in Auslassrichtung AR kontinuierlich verjüngenden und/oder verringernden Außenumfang und/oder Außendurchmesser aufweist, und
wobei der Adapter 4 mindestens einen, vorzugsweise genau einen, sich in Auslassrichtung AR unmittelbar an den ersten Abschnitt A₁ anschließenden zweiten, zylindrischen Abschnitt A₂ mit einem gegenüber dem ersten Abschnitt A₁ verringerten Außenumfang und/oder Außendurchmesser aufweist,
wobei sich der zweite Abschnitt A₂ hin zu einer Auslassöffnung 4a des Adapters 4 erstreckt und die Auslassöffnung 4a am freien Ende des zweiten Abschnitts A₂ ausgebildet und/oder vorgesehen ist,
wobei sich der erste Abschnitt A₁ ausgehend von einem maximalen Außendurchmesser Dₘₐₓ₁ des ersten Abschnitts A₁ in Auslassrichtung AR zu einem minimalen Außendurchmesser Dₘᵢₙ₁ des ersten Abschnitts A₁ verjüngt und das Verhältnis des maximalen Außendurchmessers Dₘₐₓ₁ des ersten Abschnitts A₁ zu dem minimalen Außendurchmesser Dₘᵢₙ₁ des ersten Abschnitts A₁ in einem Bereich von 1,1 bis 1,6 liegt,
wobei am Übergang vom ersten Abschnitt A₁ zum zweiten Abschnitt A₂ eine Stufe 7 ausgebildet und/oder angeordnet ist, wobei der Adapter 4 genau eine Stufe 7 am Übergang vom ersten Abschnitt A₁ zum zweiten Abschnitt A₂ aufweist,
wobei wenigstens ein dem ersten Abschnitt A₁ unmittelbar vorgelagerter dritter Abschnitt A₃ des Adapters 4 vorgesehen ist,
wobei der dritte Abschnitt A₃ einen sich in Auslassrichtung AR kontinuierlich verjüngenden und/oder verringernden Außenumfang und/oder Außendurchmesser aufweist, wobei der dritte Abschnitt A₃ stufenfrei in den ersten Abschnitt A₁ übergeht und
wobei das Verhältnis eines maximalen Außendurchmessers Dₘₐₓ₃ des dritten Abschnitts A₃ zu einem minimalen Außendurchmesser Dₘᵢₙ₃ des dritten Abschnitts A₃ in einem Bereich von 1,01 bis 1,06 liegt.

Wie insbesondere in Fig. 2 und 3 verdeutlicht, sind die Abschnitte A₁ und A₂ spezifisch zueinander ausgebildet bzw. dimensioniert, wie nachfolgend noch im Detail erläutert werden wird.

Wie insbesondere in Fig. 1 verdeutlicht, weist der Adapter 4 eine Auslassöffnung 4a, insbesondere am freien Ende des Adapters 4, auf. Die Auslassöffnung 4a dient im Anwendungs- bzw. Verwendungszustand insbesondere dem Austragen der oxybutyninhaltigen Zusammensetzung Z, und zwar insbesondere zu Zwecken der Instillation der oxybutyninhaltigen Zusammensetzung Z in die Harnblase, vorzugsweise mittels einer an die erfindungsgemäße Kolbenspritze 1 bzw. an den Adapter 4 angeschlossenen Instillationsvorrichtung.

Es kann jedoch auch zweckmäßig sein, den Spritzenzylinder 3 über die Auslassöffnung 4a zu befüllen.

Zudem veranschaulichen die vorangehenden Figurendarstellungen auch, dass der Adapter 4 eine Einlassöffnung 4b, insbesondere im Übergangsbereich zwischen Adapter 4 und Spritzenzylinder 3, aufweist. Die Einlassöffnung 4b fungiert insbesondere als Verbindung zu dem Spritzenzylinder 3 als Aufnahmeraum für die oxybutyninhaltige Zusammensetzung Z, so dass bei der Anwendung bzw. Verwendung der Kolbenspritze 1 nach der Erfindung mit Betätigung bzw. Einschieben des Spritzenkolbens 5 in einer Betätigungsrichtung die oxybutyninhaltige Zusammensetzung Z aus dem Spritzenzylinder 3 über die Einlassöffnung 4b in den Adapter 4 überführt und über die Auslassöffnung 4a ausgetragen wird.

Die Figurendarstellungen gemäß Fig. 1 bis Fig. 4 verdeutlichen zudem, dass der Adapter 4 hohl ausgebildet ist. Insbesondere weist der Adapter 4 einen entsprechenden Aufnahmeraum für die oxybutyninhaltige Zusammensetzung Z auf. Der Hohlraum des Adapters 4 steht dabei mit einem Aufnahmeraum des Spritzenzylinders 3 über die Einlassöffnung 4b in Verbindung.

Erfindungsgemäß verhält es sich insbesondere derart, dass, wie anhand der Figuren schematisch veranschaulicht, der Adapter 4 einen zwischen der Auslassöffnung 4a des Adapters 4 und der Einlassöffnung 4b des Adapters 4 befindlichen Hohlraum, insbesondere einen Durchlasskanal 9, insbesondere für den Auslass der oxybutyninhaltigen Zusammensetzung Z, aufweist. Dabei ist es insbesondere vorgesehen, dass sich der Hohlraum, insbesondere Durchlasskanal 9, von der Einlassöffnung 4b des Adapters 4 zu der Auslassöffnung 4a des Adapters 4 erstreckt bzw. die Einlassöffnung 4b mit der Auslassöffnung 4a verbindet.

Fig. 1 bis Fig. 4 und Fig. 5 verdeutlichen zudem, dass der Adapter 4 in Auslassrichtung AR bzw. in Richtung der Auslassöffnung 4a des Adapters 4 verjüngt und/oder zulaufend ausgebildet ist. Die verjüngende bzw. zulaufende Ausbildung des Adapters 4 bezieht sich dabei insbesondere auf die äußere Dimension und/oder auf die äußere Größe, bevorzugt auf den Außendurchmesser, des Adapters 4.

Erfindungsgemäß verhält es sich somit insbesondere derart, dass der Adapter 4 in Auslassrichtung AR bzw. in Richtung der Auslassöffnung 4a des Adapters 4 einen abnehmenden bzw. sich verringernden Umfang, insbesondere Außenumfang, und/oder einen abnehmenden bzw. sich verringernden Durchmesser, insbesondere Außendurchmesser, aufweist. Hierdurch ist eine besonders gute Fixierung bzw. Befestigung einer entsprechenden Instillationsvorrichtung möglich, welche beispielsweise mittels eines an der Instillationsvorrichtung angebrachten (Gegen-) Adapters an den Adapter 4 der erfindungsgemäßen Kolbenspritze 1 fixiert werden kann. Insbesondere können hierdurch auch verschiedene Instillationsvorrichtungen bzw. -schläuche mit unterschiedlichen (Innen-)Durchmessern sozusagen universell befestigt werden.

**In** diesem Zusammenhang kann ein Durchlassdurchmesser d des Durchlasskanals 9 der erfindungsgemäßen Kolbenspritze 1 gemäß einer bevorzugten Ausführungsform der vorliegenden Erfindung wie folgt ausgebildet sein (vgl. auch Fig. 3):
- Insbesondere kann der Durchlassdurchmesser d des Durchlasskanals 9 in Bezug auf eine Gesamtlänge I des Adapters 4 über mindestens 70 %, vorzugsweise mindestens 75 %, insbesondere mindestens 80 %, unverändert und/oder konstant ausgebildet sein.
- Insbesondere kann der Durchlassdurchmesser d des Durchlasskanals 9 in Bezug auf eine Gesamtlänge I des Adapters 4 über höchstens 99 %, vorzugsweise höchstens 95 %, insbesondere höchstens 90 %, unverändert und/oder konstant ausgebildet sein.
- In diesem Zusammenhang kann der Durchlassdurchmesser d des Durchlasskanals 9 in Bezug auf eine Gesamtlänge I des Adapters 4 in einem Bereich von 70 % bis 99 %, vorzugsweise in einem Bereich von 75 % bis 95 %, insbesondere in einem Bereich von 80 % bis 90 %, unverändert und/oder konstant ausgebildet sein.

Während sich also der Adapter 4 in Bezug auf seinen Außenumfang in Auslassrichtung AR verjüngt, ist der Durchlassdurchmesser d des Durchlasskanals 9 über weite Teile der Länge l des Adapters 4 konstant und/oder gleichbleibend. Dies ist einem homogenen Austrag der zu applizierenden Flüssigkeit zuträglich, wobei gleichzeitig auch eine Reduzierung bzw. Vermeidung des Todraumvolumens innerhalb des Adapters 4 erzielt wird.

Besonders bevorzugt ist der Durchlasskanal 9 über die komplette Länge l₁ des ersten Abschnitts A₁ konstant und/oder unverändert ausgebildet.

Fig. 2 und 3 verdeutlichen zudem, dass der Adapter 4 am Übergang vom ersten Abschnitt A₁ zum zweiten Abschnitt A₂ eine Stufe 7 ausgebildet und/oder angeordnet ist.

Erfindungsgemäß ist es vorgesehen, dass der Adapter 4 genau eine Stufe 7 und/oder lediglich eine (einzige) Stufe 7 am Übergang vom ersten Abschnitt A₁ zum zweiten Abschnitt A₂ aufweist.

In diesem Zusammenhang kann - ohne sich auf eine bestimmte Theorie beschränken zu wollen - sich die Ausbildung genau einer Stufe 7 zwischen dem ersten Abschnitt A₁ und dem zweiten Abschnitt A₂ als besonders vorteilhaft in Bezug auf eine hohe Dichtigkeit unter gleichzeitiger Beibehaltung einer einfachen Handhabung sowie hohen Kompatibilität erweisen. Mit anderen Worten ist der Adapter 4, mit Ausnahme der Stufe 7, stufenfrei ausgebildet, was vorteilhaft in Bezug auf eine dichtende Anlage anzuschließender Komponenten ist.

Insbesondere bei bestimmten Öffnungswinkeln, insbesondere relativ weiten Öffnungswinkeln, des weiblichen Gegenstückes kann die Stufe 7 zu einer Verbesserung der Dichtigkeit beitragen. Bei anderen, insbesondere relativ schmalen, Öffnungswinkeln kann eine Konstellation vorliegen, in welcher die Stufe 7 nicht in Kontakt mit dem Gegenstück kommt. Dann kann ab einer gewissen Länge der am Spritzenzylinder 3 angrenzende Bereich des Adapters 4, insbesondere der erste Abschnitt A₁, einen engen Kontakt zum Gegenstück bilden und die Dichtigkeit gewährleisten.

Insbesondere ist an der Stufe 7, insbesondere ein einem an den zweiten Abschnitt A₂ angrenzenden Bereich der Stufe 7, ein abgerundeter Übergang 8 vorgesehen (vgl. Fig. 2 und 3).

**In** diesem Zusammenhang kann der Übergang 8 der erfindungsgemäßen Kolbenspritze 1 gemäß einer bevorzugten Ausführungsform der vorliegenden Erfindung wie folgt ausgebildet sein:
- Insbesondere kann der Übergang 8 einen Radius von mindestens 0,1 mm, vorzugsweise mindestens 0,2 mm, insbesondere mindestens 0,3 mm, aufweisen.
- Alternativ oder zusätzlich kann der Übergang 8 einen Radius von höchstens 0,6 mm, vorzugsweise höchstens 0,5 mm, insbesondere höchstens 0,4 mm, aufweisen.
- Gemäß einer besonders bevorzugten Ausführung weist der Übergang 8 einen Radius im Bereich von 0,1 mm bis 0,6 mm, vorzugsweise im Bereich von 0,2 mm bis 0,5 mm, insbesondere im Bereich von 0,3 mm bis 0,4 mm, auf.

Wie insbesondere in Fig. 2 verdeutlicht, ist der erste Abschnitt A₁ konisch ausgebildet. Dadurch lässt sich eine sich selbst zentrierende Anordnung realisieren, wenn ein mit dem Adapter 4 zu verbindendes Bauteil auf den ersten Abschnitt A₁ aufgeschoben wird. Indem sich der erste Abschnitt A₁ in Auslassrichtung AR verjüngt, kann zudem beim Aufschieben entgegengesetzt zur Auslassrichtung AR eine Dichtkraft bzw. Presswirkung erzielt werden, einhergehend mit einer besonders dichtenden Anbindung an den Adapter 4.

Insbesondere in diesem Zusammenhang verjüngt sich der erste Abschnitt A₁ ausgehend von einem maximalen Außendurchmesser Dₘₐₓ₁ des ersten Abschnitts A₁ in Auslassrichtung AR zu einem minimalen Außendurchmesser Dₘᵢₙ₁ des ersten Abschnitts A₁.

Der erste Abschnitt A₁ der erfindungsgemäßen Kolbenspritze 1 bzw. des Adapters 4 der erfindungsgemäßen Kolbenspritze 1 kann gemäß einer bevorzugten Ausführungsform der vorliegenden Erfindung dabei wie folgt ausgebildet sein:
- Das Verhältnis des maximalen Außendurchmessers Dₘₐₓ₁ des ersten Abschnitts A₁ zu dem minimalen Außendurchmesser Dₘᵢₙ₁ des ersten Abschnitts A₁ beträgt mindestens 1,1, insbesondere mindestens 1,15, vorzugsweise mindestens 1,2, bevorzugt mindestens 1,25, besonders bevorzugt mindestens 1,3.
- Das Verhältnis des maximalen Außendurchmessers Dₘₐₓ₁ des ersten Abschnitts A₁ zu dem minimalen Außendurchmesser Dₘᵢₙ₁ des ersten Abschnitts A₁ beträgt höchstens 1,6, insbesondere höchstens 1,5, vorzugsweise höchstens 1,45, bevorzugt höchstens 1,4, besonders bevorzugt höchstens 1,35.
- Alternativ oder zusätzlich liegt das Verhältnis des maximalen Außendurchmessers Dₘₐₓ₁ des ersten Abschnitts A₁ zu dem minimalen Außendurchmesser Dₘᵢₙ₁ des ersten Abschnitts A₁ in einem Bereich von 1,1 bis 1,6, insbesondere in einem Bereich von 1,15 bis 1,5, vorzugsweise in einem Bereich von 1,2 bis 1,45, bevorzugt in einem Bereich von 1,25 bis 1,4, besonders bevorzugt in einem Bereich von 1,3 bis 1,35 .

Die obigen Verhältnisangaben in Bezug auf den minimalen Durchmesser Dₘᵢₙ₁ einerseits sowie Dₘₐₓ₁ andererseits wurden im Rahmen von umfangreichen Versuchen in Bezug auf die Dichtigkeit ermittelt und haben sich ganz besonders zweckmäßig herausgestellt.

Wie bereits zuvor erläutert, liegt ein Erfindungsgedanke der vorliegenden Erfindung darin, dass der Adapter 4 der erfindungsgemäßen Kolbenspritze 1 genau eine (einzige) Stufe 7 aufweist.

Was die Dimensionierung und/oder die Höhe dieser Stufe 7 anbelangt, so wird diese vorzugsweise durch das Verhältnis des minimalen Durchmessers Dₘᵢₙ₁ des ersten Abschnitts A₁ einerseits zu dem Durchmesser D₂, insbesondere Außendurchmesser, des zweiten Abschnitts A₂ andererseits definiert.

Die gezielte Einstellung dieses Verhältnisses zur Dimensionierung bzw. Ausbildung der Stufe 7 hat sich dabei als besonders zweckmäßig herausgestellt, insbesondere im Hinblick auf eine feste Anbindung an eine Instillationsvorrichtung sowie eine sichere und zuverlässig abdichtende Anschlussgeometrie.

In Bezug auf das in Rede stehende Verhältnis zur Ausbildung der Stufe 7 kann es sich erfindungsgemäß insbesondere wie folgt verhalten:
- Insbesondere kann das Verhältnis des minimalen Außendurchmessers Dₘᵢₙ₁ des ersten Abschnitts A₁ zu dem Außendurchmesser D₂ des zweiten Abschnitts A₂ mindestens 1,1, insbesondere mindestens 1,2, vorzugsweise mindestens 1,3, bevorzugt mindestens 1,4, insbesondere mindestens 1,5, betragen.
- Alternativ oder zusätzlich kann das Verhältnis des minimalen Außendurchmessers Dₘᵢₙ₁ des ersten Abschnitts A₁ zu dem Außendurchmesser D₂ des zweiten Abschnitts A₂ höchstens 2, insbesondere höchstens 1,9, vorzugsweise höchstens 1,8, bevorzugt höchstens 1,7, insbesondere höchstens 1,6, betragen.
- Gemäß einer besonders bevorzugten Ausführungsform der vorliegenden Erfindung kann das Verhältnis des minimalen Außendurchmessers Dₘᵢₙ₁ des ersten Abschnitts A₁ zu dem Außendurchmesser D₂ des zweiten Abschnitts A₂ in einem Bereich von 1,1 bis 2, insbesondere in einem Bereich von 1,2 bis 1,9, vorzugsweise in einem Bereich von 1,3 bis 1,8, bevorzugt in einem Bereich von 1,4 bis 1,7, insbesondere in einem Bereich von 1,5 bis 1,6, liegen.

Durch die gezielte Dimensionierung der Stufe 7 konnte im Rahmen praktischer Erprobungen ein optimaler Kompromiss zwischen hoher Dichtigkeit, einfacher Handhabung sowie hoher Kompatibilität erzielt werden. In diesem Zusammenhang ist die Dimensionierung der Stufe 7 auch im Hinblick auf ein Zusammenwirken eines zugeordneten Verschlusselementes 13 von hoher Bedeutung, wie nachfolgend noch im Detail erläutert werden wird.

Eine weitere wichtige Kenngröße in Bezug auf den erfindungsgemäß konzipierten Adapter 4 liegt vorzugsweise in einer Abstimmung eines maximalen Durchmessers Dₘₐₓ₁ des ersten Abschnitts A₁ einerseits zu dem Durchmesser D₂ des zweiten Abschnitts A₂ andererseits. Diesbezüglich verhält es sich erfindungsgemäß insbesondere wie folgt:
- Insbesondere kann das Verhältnis des maximalen Außendurchmessers Dₘₐₓ₁ des ersten Abschnitts A₁ des Adapters 4 zu dem Außendurchmesser D₂ des zweiten Abschnitts A₂ des Adapters 4 mindestens 1,7, insbesondere mindestens 1,8, vorzugsweise mindestens 1,9, bevorzugt mindestens 2, betragen.
- Alternativ oder zusätzlich kann das Verhältnis des maximalen Außendurchmessers Dₘₐₓ₁ des ersten Abschnitts A₁ des Adapters 4 zu dem Außendurchmesser D₂ des zweiten Abschnitts A₂ des Adapters höchstens 2,5, insbesondere höchstens 2,4, vorzugsweise höchstens 2,3, bevorzugt höchstens 2,2, betragen.
- Besonders bevorzugt kann das Verhältnis des maximalen Außendurchmessers Dₘₐₓ₁ des ersten Abschnitts A₁ des Adapters 4 zu dem Außendurchmesser D₂ des zweiten Abschnitts A₂ des Adapters 4 in einem Bereich von 1,7 bis 2,5, insbesondere in einem Bereich von 1, 8 bis 2,4, vorzugsweise in einem Bereich von 1,9 bis 2,3, bevorzugt in einem Bereich von 2 bis 2,2, liegen.

Wie Fig. 1, 2, 3, 4 und Fig. 5 gleichermaßen veranschaulichen, kann sich der zweite Abschnitt A₂, vorzugsweise ausgehend von dem minimalen Durchmesser Dₘᵢₙ₁, insbesondere minimalen Außendurchmesser, des ersten Abschnitts A₁, hin zu der Auslassöffnung 4a des Adapters 4 erstrecken.

Insbesondere ist die Auslassöffnung 4a am freien Ende des zweiten Abschnitts A₂ ausgebildet und/oder vorgesehen.

Wie insbesondere anhand von Fig. 2 ersichtlich, weist der zweite Abschnitt A₂ zur Ausbildung der Auslassöffnung 4a einen vorzugsweise radial nach innen in einen Durchlasskanal 9 des Adapters 4 ragenden, vorzugsweise umlaufenden, Vorsprung 10 auf.

Diesbezüglich kann es sich erfindungsgemäß insbesondere wie folgt verhalten (vgl. insbesondere Fig. 2):
- Insbesondere ist ein durch den Vorsprung 10 gebildeter Auslassdurchmesser dₐᵤₛ der Auslassöffnung 4a gegenüber einem durch den Durchlasskanal 9 gebildeten Durchlassdurchmesser d reduziert.
- Alternativ oder zusätzlich beträgt das Verhältnis des Durchmessers D₂, insbesondere Außendurchmessers, des zweiten Abschnitts A₂ zu dem durch den Vorsprung 10 gebildeten Auslassdurchmesser dₐᵤₛ, insbesondere Innendurchmesser, der Auslassöffnung 4a mindestens 1,25, insbesondere mindestens 1,5, vorzugsweise mindestens 2, bevorzugt mindestens 2,5.
- Besonders bevorzugt beträgt das Verhältnis des Durchmessers D₂, insbesondere Außendurchmessers, des zweiten Abschnitts A₂ zu dem durch den Vorsprung 10 gebildeten Auslassdurchmesser dₐᵤₛ, insbesondere Innendurchmesser, der Auslassöffnung 4a höchstens 3,5, insbesondere höchstens 3,25, vorzugsweise höchstens 3, bevorzugt höchstens 2,75.
- Insbesondere liegt das Verhältnis des Durchmessers D₂, insbesondere Außendurchmessers, des zweiten Abschnitts A₂ zu dem durch den Vorsprung 10 gebildeten Auslassdurchmesser dₐᵤₛ, insbesondere Innendurchmesser, der Auslassöffnung 4a in einem Bereich von 1,25 bis 3,5, vorzugsweise in einem Bereich von 1,5 bis 3,25, bevorzugt in einem Bereich von 2 bis 3, insbesondere in einem Bereich von 2,5 bis 2,75.

Erfindungsgemäß verhält es sich in Bezug auf den vorzugsweise vorgesehenen Vorsprung 10 dabei insbesondere derart, dass infolge des Vorsprungs 10 ein Auslassdurchmesser dₐᵤₛ gebildet ist, der gegenüber einem Durchlassdurchmesser d des zugeordneten Durchlasskanals 9 reduziert ist. Durch die Reduzierung des Durchlassdurchmesser d im Bereich des Vorsprungs 10 kann sich strömungstechnisch eine Beschleunigung des auszutragenden Fluides einstellen, was den Austrag begünstigt.

Das diesbezügliche Verhältnis des Durchlassdurchmessers d zu dem Auslassdurchmesser dₐᵤₛ kann in diesem Zusammenhang insbesondere wie folgt ausgebildet sein:
- Insbesondere beträgt das Verhältnis des Durchlassdurchmessers d zu dem reduzierten und/oder durch den Vorsprung 10 gebildeten Auslassdurchmesser dₐᵤₛ mindestens 1,3, insbesondere mindestens 1,4, vorzugsweise mindestens 1,5, bevorzugt mindestens 1,6.
- Alternativ oder zusätzlich kann das Verhältnis des Durchlassdurchmessers d zu dem durch den Vorsprung 10 gebildeten Auslassdurchmesser dₐᵤₛ höchstens 2, insbesondere höchstens 1,9, vorzugsweise höchstens 1,8, bevorzugt höchstens 1,7, betragen.
- Insbesondere kann das Verhältnis des Durchmessers des Durchlassdurchmessers d zu dem durch den Vorsprung 10 gebildeten Auslassdurchmesser dₐᵤₛ im Bereich von 1,3 bis 2, vorzugsweise im Bereich von 1,4 bis 1,9, insbesondere im Bereich von 1,5 bis 1,8, bevorzugt im Bereich von 1,6 bis 1,7, liegen.

Wie insbesondere in Fig. 2 schematisiert ist, ist am freien Ende des zweiten Abschnitts A₂ und/oder an die Auslassöffnung 4a angrenzend eine vorzugsweise umlaufende Rundung 11 vorgesehen und/oder ausgebildet. Dies ermöglicht eine dichtende Auflage, insbesondere mit einem auf den zweiten Abschnitt A₂ optional aufzusetzenden Verschlusselement 13.

Die vorzugsweise umlaufende Rundung 11 kann in diesem Zusammenhang insbesondere wie folgt ausgebildet sein:
- Insbesondere kann die Rundung 11 einen Radius von mindestens 0,8 mm, vorzugsweise mindestens 1 mm, insbesondere mindestens 1,2 mm, besonders bevorzugt mindestens 1,4 mm, aufweisen.
- Alternativ oder zusätzlich kann die Rundung 11 einen Radius von höchstens 2,2 mm, vorzugsweise höchstens 2 mm, insbesondere höchstens 1,8 mm, besonders bevorzugt höchstens 1,6 mm aufweisen.
- Besonders bevorzugt kann die Rundung 11 einen Radius im Bereich von 0,8 mm bis 2,2 mm, vorzugsweise im Bereich von 1 mm bis 2 mm, insbesondere im Bereich von 1,2 mm bis 1,8 mm, besonders bevorzugt im Bereich von 1,4 mm bis 1,6 mm, aufweisen.

Was die endseitige Ausbildung des zweiten Abschnitts A₂ weiterführend anbelangt, so kann der zweite Abschnitt A₂ vorzugsweise umlaufend an der Auslassöffnung 4a und/oder an der Auslassöffnung 4a angrenzend angefast sein. Durch die auslassseitige Anfasung der Auslassöffnung 4a lässt sich strömungstechnisch ein besonders zweckmäßiger Austritt der zu verabreichenden Substanz bewerkstelligen.

Wie insbesondere in den Fig. 2 und 3 verdeutlicht, ist wenigstens ein, vorzugsweise genau ein, dem ersten Abschnitt A₁ unmittelbar vorgelagerter dritter Abschnitt A₃ des Adapters 4 vorgesehen.

Der dritte Abschnitt A₃ weist einen sich in Auslassrichtung AR kontinuierlich verjüngenden und/oder verringernden Außenumfang Außendurchmesser auf.

Der vorgesehene dritte Abschnitt A₃ verstärkt insbesondere die zur Verfügung stehende Dichtfläche und erhöht somit eine sichere Anbindung an zugeordnete Katheter oder sonstige Vorrichtungen im Rahmen der Verabreichung einer Substanz, insbesondere in die Harnblase.

Insbesondere weist der dritte Abschnitt A₃ mit seiner spezifischen Ausbildung ein hohes Maß an Kompatibilität mit einer Vielzahl von Kathetern bzw. Katheteranschlüssen, vorzugsweise mit sämtlichen auf dem Markt verfügbaren Kathetern bzw. Katheteranschlüssen, auf.

Der dritte Abschnitt A₃ geht stufenfrei in den ersten Abschnitt A₁ über.

Besonders bevorzugt weisen der dritte Abschnitt A₃ und der erste Abschnitt A₁ an ihrem gemeinsamen Übergang denselben Durchmesser, insbesondere Außendurchmesser, auf.

Insbesondere ist in diesem Zusammenhang auch vorgesehen, dass der dritte Abschnitt A₃ unmittelbar angrenzend an den Spritzenkörper 2, vorzugsweise an den Spritzenzylinder 3, ausgebildet und/oder angeordnet ist.

Was Ausbildung und/oder Dimensionierung des dritten Abschnitts A₃ weiterführend anbelangt, so haben sich insbesondere folgende Ausbildungen bzw. Dimensionierung als zweckmäßig erwiesen:
- Das Verhältnis des maximalen Außendurchmessers Dₘₐₓ₃ des dritten Abschnitts A₃ zu dem minimalen Außendurchmesser Dₘᵢₙ₃ des dritten Abschnitts A₃ beträgt mindestens 1,01, insbesondere mindestens 1,02, vorzugsweise mindestens 1,03, bevorzugt mindestens 1,035 .
- Das Verhältnis des maximalen Außendurchmessers Dₘₐₓ₃ des dritten Abschnitts A₃ zu dem minimalen Außendurchmesser Dₘᵢₙ₃ des dritten Abschnitts A₃ beträgt höchstens 1,06, insbesondere höchstens 1,05, vorzugsweise höchstens 1,045, bevorzugt höchstens 1,04 .
- Das Verhältnis des maximalen Außendurchmessers Dₘₐₓ₃ des dritten Abschnitts A₃ zu dem minimalen Außendurchmesser Dₘᵢₙ₃ des dritten Abschnitts A₃ liegt in einem Bereich von 1,01 bis 1,06, insbesondere in einem Bereich von 1,02 bis 1,05, vorzugsweise in einem Bereich von 1,03 bis 1,045, bevorzugt in einem Bereich von 1,035 bis 1,04 .

Was, insbesondere unter Bezugnahme auf Fig. 2, das Verhältnis des maximalen Durchmessers Dₘₐₓ₃ des dritten Abschnitts A₃ zu dem minimalen Durchmesser Dₘᵢₙ₁ des ersten Abschnitts A₁ weiterführend anbelangt, so verhält es sich erfindungsgemäß insbesondere wie folgt:
- Insbesondere beträgt das Verhältnis des maximalen Außendurchmessers Dₘₐₓ₃ des dritten Abschnitts A₃ zu dem minimalen Außendurchmesser Dₘᵢₙ₁ des ersten Abschnitts A₁ und/oder des Durchmessers des ersten Abschnitts A₁ an der Stufe 7 des Adapters 4 mindestens 1,05, insbesondere mindestens 1,15, vorzugsweise mindestens 1,25, bevorzugt mindestens 1,35.
- Vorzugsweise beträgt das Verhältnis des maximalen Außendurchmessers Dₘₐₓ₃ des dritten Abschnitts A₃ zu dem minimalen Außendurchmesser Dₘᵢₙ₁ des ersten Abschnitts A₁ und/oder des Durchmessers an der Stufe 7 des Adapters 4 höchstens 1,75, insbesondere mindestens 1,65, vorzugsweise mindestens 1,55, bevorzugt mindestens 1,45.
- Besonders bevorzugt liegt das Verhältnis des maximalen Außendurchmessers Dₘₐₓ₃ des dritten Abschnitts A₃ zu dem minimalen Außendurchmesser Dₘᵢₙ₁ des ersten Abschnitts A₁ und/oder des Durchmessers an der Stufe 7 des Adapters 4 in einem Bereich von 1,05 bis 1,75, insbesondere in einem Bereich von 1,15 bis 1,65, vorzugsweise in einem Bereich von 1,25 bis 1,55, bevorzugt in einem Bereich von 1,35 bis 1,45.

Was, insbesondere unter Bezugnahme auf Fig. 2, das Verhältnis des maximalen Durchmessers Dₘₐₓ₃ des dritten Abschnitts A₃ zu dem Durchmesser D₂ des zweiten Abschnitts A₂ weiterführend anbelangt, so verhält es sich erfindungsgemäß insbesondere wie folgt:
- Insbesondere beträgt das Verhältnis eines maximalen Außendurchmessers Dₘₐₓ₃ des dritten Abschnitts A₃ des Adapters 4 zu dem Außendurchmesser D₂ des zweiten Abschnitts A₂ des Adapters 4 mindestens 1,7, insbesondere mindestens 1,8, vorzugsweise mindestens 1,9, bevorzugt mindestens 2.
- Besonders bevorzugt beträgt das Verhältnis eines maximalen Außendurchmessers Dₘₐₓ₃ des dritten Abschnitts A₃ des Adapters 4 zu dem Außendurchmesser D₂ des zweiten Abschnitts A₂ des Adapters 4 höchstens 2,5, insbesondere höchstens 2,4, vorzugsweise höchstens 2,3, bevorzugt höchstens 2,2.
- Ganz besonders bevorzugt liegt das Verhältnis eines maximalen Außendurchmessers Dₘₐₓ₃ des dritten Abschnitts A₃ des Adapters 4 zu dem Außendurchmesser D₂ des zweiten Abschnitts A₂ des Adapters 4 in einem Bereich von 1,7 bis 2,5, insbesondere in einem Bereich von 1,8 bis 2,4, vorzugsweise in einem Bereich von 1,9 bis 2,3, bevorzugt in einem Bereich von 2 bis 2,2.

Erfindungsgemäß hat es sich als zweckmäßig und bevorzugt erwiesen, wenn im Bereich der Einlassöffnung 4b des Durchlasskanals 9 und/oder im Übergangsbereich zwischen Adapter 4 und Spritzenzylinder 3 ein abgerundeter Übergang 12 vorgesehen ist (vgl. Fig. 2).

Mit anderen Worten ermöglicht der Übergang 12 somit eine abgerundete Eintrittspassage des zu verabreichenden Fluids in den Adapter 4, was strömungstechnisch vorteilhaft ist. Auch spritzgusstechnisch ist eine derartige Ausbildung vorteilhaft, da die Wanddicke ein bestimmtes, praktikables Maß nicht übersteigt.

In Bezug auf den vorzugsweise vorgesehenen Übergang 12 verhält es sich dabei insbesondere wie folgt:
- Insbesondere kann der Übergang 12 einen Radius von mindestens 1 mm, vorzugsweise mindestens 2 mm, insbesondere mindestens 3 mm, aufweisen.
- Vorzugsweise weist der Übergang 12 einen Radius von höchstens 6 mm, vorzugsweise höchstens 5 mm, insbesondere höchstens 4 mm, auf.
- Besonders bevorzugt weist der Übergang 12 einen Radius im Bereich von 1 mm bis 6 mm, vorzugsweise im Bereich von 2 mm bis 5 mm, insbesondere im Bereich von 3 mm bis 4 mm, auf.

Wie insbesondere anhand von Fig. 3 ersichtlich, können den in Rede stehenden Abschnitten A₁, A₂ und A₃ entsprechend zugeordnete Längenangaben bzw. Längenabschnitte I₁, I₂ sowie I₃ zugeordnet werden.

Die nachfolgend wiedergegebenen, bevorzugten Längsverhältnisse wurden in diesem Zusammenhang im Rahmen von praktischen Erprobungen ermittelt und begünstigen die erfindungsgemäße Zielsetzung einer dichtenden, komfortablen und eine hohe Kompatibilität aufweisenden Anbindung des Adapters 4.

Nachfolgend werden somit, insbesondere in Bezug auf Fig. 3, dementsprechend bevorzugte Verhältnisangaben und Ausgestaltungen erläutert.

Hinsichtlich des Verhältnisses der Länge I₁ des ersten Abschnitts A₁ des Adapters 4 zu der Länge I₂ des zweiten Adapters A₂ des Adapters 4 verhält es sich in diesem Zusammenhang vorzugsweise wie folgt:
- Insbesondere beträgt das Verhältnis der Länge I₁ des ersten Abschnitts A₁ des Adapters 4 zu der Länge I₂ des zweiten Abschnitts A₂ des Adapters 4 mindestens 1,1, insbesondere mindestens 1,2, vorzugsweise mindestens 1,3, bevorzugt mindestens 1,4.
- Vorzugsweise beträgt das Verhältnis der Länge I₁ des ersten Abschnitts A₁ des Adapters 4 zu der Länge I₂ des zweiten Abschnitts A₂ des Adapters 4 höchstens 1,8, insbesondere höchstens 1,7, vorzugsweise höchstens 1,6, bevorzugt höchstens 1,5.
- Gemäß einer besonders bevorzugten Ausführungsform liegt das Verhältnis der Länge I₁ des ersten Abschnitts A₁ des Adapters 4 zu der Länge I₂ des zweiten Abschnitts A₂ des Adapters 4 in einem Bereich von 1,1 bis 1,8, insbesondere in einem Bereich von 1,2 bis 1,7, vorzugsweise in einem Bereich von 1,3 bis 1,6, bevorzugt in einem Bereich von 1,4 bis 1,5.

Dementsprechend ist der erste Abschnitt A₁ signifikant länger als der zweite Abschnitt A₂ ausgebildet. Dadurch wird über den ersten Abschnitt A₁ ein entsprechend länglicher Dichtabschnitt ausgebildet, was bevorzugt im Sinne der vorliegenden Erfindung eine Hauptfunktion des ersten Abschnitts A₁ darstellt.

Weiterführend kann es sich in Bezug auf die Längenangaben der einzelnen Abschnitte A₁, A₂ sowie A₃ insbesondere wie folgt verhalten:
- Insbesondere beträgt das Verhältnis der Länge I₁ des ersten Abschnitts A₁ des Adapters 4 zu der Länge I₃ des dritten Abschnitts A₃ des Adapters 4 mindestens 1,6, insbesondere mindestens 1,7, vorzugsweise mindestens 1,8, bevorzugt mindestens 1,9.
- Bevorzugt beträgt das Verhältnis der Länge I₁ des ersten Abschnitts A₁ des Adapters 4 zu der Länge I₃ des dritten Abschnitts A₃ des Adapters 4 höchstens 2,4, insbesondere höchstens 2,3, insbesondere höchstens 2,2, vorzugsweise höchstens 2,1.
- Alternativ oder zusätzlich liegt das Verhältnis der Länge I₁ des ersten Abschnitts A₁ des Adapters 4 zu der Länge I₃ des dritten Abschnitts A₃ des Adapters 4 in einem Bereich von 1,6 bis 2,4, insbesondere in einem Bereich von 1,7 bis 2,3, vorzugsweise in einem Bereich von 1,8 bis 2,2, bevorzugt in einem Bereich von 1,9 bis 2,1.
- Insbesondere beträgt das Verhältnis der Länge I₂ des zweiten Abschnitts A₂ des Adapters 4 zu der Länge I₃ des dritten Abschnitts A₃ des Adapters 4 mindestens 1,1, insbesondere mindestens 1,2, vorzugsweise mindestens 1,3, bevorzugt mindestens 1,4.
- Vorzugsweise beträgt das Verhältnis der Länge I₂ des zweiten Abschnitts A₂ des Adapters 4 zu der Länge I₃ des dritten Abschnitts A₃ des Adapters 4 höchstens 1,8, vorzugsweise höchstens 1,7, bevorzugt höchstens 1,6, besonders bevorzugt höchstens 1,5.
- Alternativ oder zusätzlich liegt das Verhältnis der Länge I₂ des zweiten Abschnitts A₂ des Adapters 4 zu der Länge I₃ des dritten Abschnitts A₃ des Adapters 4 in einem Bereich von 1,1 bis 1,8, insbesondere in einem Bereich von 1,2 bis 1,7, vorzugsweise in einem Bereich von 1,3 bis 1,6, bevorzugt in einem Bereich von 1,4 bis 1,5.

Mit anderen Worten verhält es sich erfindungsgemäß vorzugsweise somit derart, dass der erste Abschnitt A₁ länger als der zweite Abschnitt A₂ und/oder der dritte Abschnitt A₃ ist. In diesem Zusammenhang ist es besonders bevorzugt, dass der zweite Abschnitt A₂ länger als der optional vorgesehene dritte Abschnitt A₃ ist.

Dadurch lassen sich entsprechende Vorteile und Besonderheiten, insbesondere im Hinblick auf eine hohe Abdichtung, zuverlässige Handhabung sowie hohe Kompatibilität, erzielen.

Weiterhin veranschaulichen die Figurendarstellungen gemäß Fig. 4, 5 und 6 eine erfindungsgemäß bevorzugte Ausführungsform, wonach die Kolbenspritze 1 ein insbesondere abnehmbares bzw. entfernbares und/oder lösbares Verschlusselement 13 vorzugsweise zum insbesondere abdichtenden Anliegen an den zweiten Abschnitt A₂ des Adapters 4, und/oder vorzugsweise zum insbesondere abdichtenden Aufsetzen auf den zweiten Abschnitt A₂ des Adapters 4 und/oder vorzugsweise zum abdichtenden Verschließen der Auslassöffnung 4a des Adapters 4, aufweist.

Mit anderen Worten ist erfindungsgemäß vorzugsweise vorgesehen, dass der Adapter 4, insbesondere die Auslassöffnung 4a, mit dem Verschlusselement 13 verschlossen ist.

In diesem Zusammenhang ist insbesondere vorgesehen, dass das Verschlusselement 13 den zweiten Abschnitt A₂ des Adapters 4, insbesondere mitsamt der Auslassöffnung 4a, zumindest im Wesentlichen vollständig und/oder abdichtend umschließt und/oder abdeckt, insbesondere zumindest im Wesentlichen über die gesamte Außenfläche und/oder (Außen-)Wandung des zweiten Abschnitts A₂ des Adapters 4 und/oder insbesondere zumindest im Wesentlichen über die gesamte Auslassöffnung 4a des Adapters 4.

Das Verschlusselement 13 ist somit vorzugsweise zum insbesondere abdichtenden Anliegen an einer zwischen dem ersten Abschnitt A₁ und dem zweiten Abschnitt A₂ ausgebildeten Stufe 7 ausgebildet.

Insbesondere ist die Stufe 7 als axialer Anschlag für das Verschlusselement 13 ausgebildet und/oder ist das Verschlusselement 13 kappen- und/oder hutförmig ausgebildet.

Besonders bevorzugt weist das Verschlusselement 13 einen vorzugsweise zum zweiten Abschnitt A₂ komplementären Aufnahmeraum, insbesondere zur zumindest im Wesentlichen vollständigen Aufnahme und/oder zum zumindest im Wesentlichen vollständigen Umschließen des zweiten Abschnitts A₂ des Adapters 4, auf.

In diesem Zusammenhang ist insbesondere vorgesehen, dass das Verschlusselement 13 form- und/oder kraftschlüssig, insbesondere reibschlüssig, an dem zweiten Abschnitt A₂ des Adapters 4, anliegt und/oder anliegbar ausgebildet ist und/oder dass das Verschlusselement 13 den zweiten Abschnitt A₂ des Adapters 4, form- und/oder kraftschlüssig, insbesondere reibschlüssig, umschließt und/oder umschließbar ausgebildet ist.

Mit anderen Worten verhält es sich erfindungsgemäß in Bezug auf das Verschlusselement 13 insbesondere derart, dass das Verschlusselement 13 ausschließlich mit dem zweiten Abschnitt A₂ in Kontakt tritt und/oder zur dichtenden Anlage gebracht wird, insbesondere wobei das Verschlusselement 13 auf den zweiten Abschnitt A₂ aufschiebbar und nach dem Aufschieben axial gegen die Stufe 7 anschlagbar ist. Während somit der erste Abschnitt A₁ insbesondere zur dichtenden Anlage einer zugeordneten Instillationsvorrichtung und/oder eines Katheter sowie einer sonstigen Verabreichungsvorrichtung bzw. -leitung konzipiert ist, fungiert der zweite Abschnitt A₂ vornehmlich zum Zusammenwirken mit einem Verschlusselement 13, insbesondere zum dichtenden Verschließen der Kolbenspritze 1 bzw. des Adapters 4 nach und/oder vor der Verabreichung.

Was das Verschlusselement 13 weiterführend anbelangt, so ist vorzugsweise vorgesehen, dass das Verschlusselement 13 aus einem von dem Spritzenkörper 2, insbesondere aus einem von dem Adapter 4 und/oder dem Spritzenzylinder 3, vorzugsweise aus einem von dem Adapter 4, verschiedenem Material gebildet ist.

Insbesondere verhält es sich erfindungsgemäß vorzugsweise derart, dass das Verschlusselement 13 aus einem weichen und/oder elastischen und/oder reversibel dehnbaren und/oder abdichtendem Material gebildet ist.

Besonders bevorzugt ist das Verschlusselement 13 aus einem elastomeren Material, insbesondere aus einem Gummimaterial und/oder Kautschukmaterial, gebildet.

In diesem Zusammenhang hat es sich als besonders vorteilhaft herausgestellt, dass das Verschlusselement 13 aus einem Halogenbutylkautschuk, insbesondere aus einem Chlorbutylkautschuk oder aus einem Brombutylkautschuk, vorzugsweise aus einem Brombutylkautschuk, gebildet ist.

Insbesondere die Figur 5 verdeutlicht in diesem Zusammenhang gleichermaßen eine erfindungsgemäß bevorzugte Ausführungsform, wonach das Verschlusselement 13 einen vorzugsweise zylindrischen Grundkörper 14 aufweist, in welchem der Aufnahmeraum ausgebildet ist, insbesondere wobei der Aufnahmeraum vorzugsweise zumindest im Wesentlichen komplementär zum zweiten Abschnitt A₂ und/oder zur vorzugsweise zumindest im Wesentlichen passgenauen und/oder abdichtenden Aufnahme des zweiten Abschnitts A₂ ausgebildet ist.

In diesem Zusammenhang ist dabei insbesondere vorgesehen, dass der Grundkörper 14 eine einseitige, mit dem Aufnahmeraum verbundene und/oder an den Aufnahmeraum angrenzende Öffnung aufweist, über welche das Verschlusselement 13 auf den zweiten Abschnitt A₂ aufbringbar und/oder aufschiebbar ist.

Dabei verhält es sich vorzugsweise derart, dass der Grundkörper 14 in einer Verschluss- und/oder Dichtposition axial gegen eine zwischen dem ersten Abschnitt A₁ und dem zweiten Abschnitt A₂ vorgesehenen und/oder ausgebildeten Stufe 7 des Adapters 4 zur Anlage bringbar und/oder anschlagbar ist.

Wie weiterführend insbesondere anhand von Fig. 5 ersichtlich, weist das Verschlusselement 13 einen tellerartigen und/oder kappenförmigen Griffabschnitt 15 auf, insbesondere wobei der Griffabschnitt 15 gegenüber dem Grundkörper 14 radial vorspringend, insbesondere umlaufend radial vorspringend, ausgebildet ist.

Dabei ist der Griffabschnitt 15 vorzugsweise an dem Grundkörper 14 angeordnet, insbesondere angeformt.

Erfindungsgemäß ist bzw. sind der Aufnahmeraum und/oder der Grundkörper 14 einseitig durch den Griffabschnitt 15 verschlossen, insbesondere wobei an einer dem Griffabschnitt 15 abgewandten Seite des Grundkörpers 14 ein Anschlag ausgebildet ist, wobei der Anschlag in einer Verschluss- und/oder Dichtposition des Verschlusselements 13 gegen eine zwischen dem ersten Abschnitt A₁ und dem zweiten Abschnitt A₂ vorgesehenen und/oder ausgebildeten Stufe 7 des Adapters 4 zur Anlage bringbar ist.

Dabei kann es zweckmäßig sein, dass der Grundkörper 14 einseitig geöffnet zum Einführen des zweiten Abschnitts A₂ in den Aufnahmeraum und auf der gegenüberliegenden Seite durch den Griffabschnitt 15 verschlossen ist.

Insbesondere sind der Grundkörper 14 und der Griffabschnitt 15 einstückig ausgebildet.

Gemäß einer besonders bevorzugten Ausführungsform der vorliegenden Erfindung ist eine der Auslassöffnung 4a zugewandte Dichtseite des Griffabschnitts 15 zum insbesondere abdichtenden Anliegen an die Auslassöffnung 4a und/oder zum Verschließen der Auslassöffnung 4a und/oder zum insbesondere abdichtenden Aufsetzen auf die Auslassöffnung 4a und/oder zum im Wesentlichen vollständigen und/oder abdichtenden Umschließen der Auslassöffnung 4a ausgebildet.

Bevorzugt ist in diesem Zusammenhang, dass an und/oder auf der Dichtseite ein in die Auslassöffnung 4a hineinreichender und/oder hineinragender, vorzugsweise gewölbter und/oder in Form einer Wölbung ausgebildeter, Dichtabschnitt 16 vorgesehen ist, insbesondere wobei der Dichtabschnitt 16 axial hervorspringend auf der Dichtseite des Griffabschnitts 15 angeformt ist.

Wie insbesondere anhand von Fig. 5 verdeutlicht, ist das Verschlusselement 13 derart auf den zweiten Abschnitt A₂ aufsteckbar und/oder aufsetzbar und/oder aufschiebbar, dass der Grundkörper 14 außenseitig zumindest im Wesentlichen glatt und/oder stufenfrei in den anschließenden ersten Abschnitt A₁ des Adapters 4 übergeht, insbesondere wobei der Durchmesser, insbesondere Außendurchmesser, des Grundkörpers 14 zumindest im Wesentlichen dem minimalen Durchmesser, insbesondere minimalen Außendurchmesser Dₘᵢₙ₁, des ersten Abschnitts A₁ entspricht.

Der glatte und/oder stufenfreie außenseitige Übergang des Verschlusselements 13 bzw. des Grundkörpers 14 zu dem anschließenden ersten Abschnitt A₁ begünstigt ebenfalls eine sichere Anbindung des Verschlusselements 13, insbesondere da kein unbeabsichtigtes Abrutschen des Verschlusselements 13 aufgrund vorspringender Abschnitte oder dergleichen erfolgt. Dementsprechend ist die Stufe 7 ganz gezielt auch im Hinblick auf die Dicke des Verschlusselements 13 bzw. des Grundkörpers 14 dimensioniert, um den stufenfreien und/oder glatten Übergang entsprechend zu realisieren.

Was die erfindungsgemäße Kolbenspritze 1 generell anbelangt, so kann es sich vorzugsweise aufgrund weiterer bevorzugter Ausführungsformen insbesondere wie folgt verhalten:
- Insbesondre ist der Spritzenkörper 2 mittels Verkleben, Verschweißen, Verschmelzen und/oder Gießen einstückig ausgebildet.
- Vorzugsweise ist die Verbindung, insbesondere die feste und/oder unlösbare Verbindung, vorzugsweise die stoffschlüssige Verbindung, des Adapters 4 mit dem Spritzenzylinder 3 mittels Verkleben, Verschweißen, Verschmelzen und/oder Gießen erzeugt.

- Alternativ oder zusätzlich ist der Adapter 4 mit dem Spritzenzylinder 3 mittels Verkleben, Verschweißen, Verschmelzen und/oder Gießen verbunden, insbesondere fest und/oder unlösbar verbunden, vorzugsweise stoffschlüssig verbunden.
- Besonders bevorzugt ist der Spritzenkörper 2 mittels Spritzgießen, Gießformen, Extrudieren und/oder Glasformen, insbesondere mittels Spritzgießen, Gießformen und/oder Extrudieren, einstückig ausgebildet.
- Was eine weitere, bevorzugte Ausgestaltung der vorliegenden Erfindung anbelangt, ist vorgesehen, dass die Verbindung, insbesondere die feste und/oder unlösbare Verbindung, vorzugsweise die stoffschlüssige Verbindung, des Adapters 4 mit dem Spritzenzylinder 3 mittels Spritzgießen, Gießformen, Extrudieren und/oder Glasformen, insbesondere mittels Spritzgießen, Gießformen und/oder Extrudieren, erzeugt ist.
- Insbesondere ist es möglich, dass der Adapter 4 mit dem Spritzenzylinder 3 mittels Spritzgießen, Gießformen, Extrudieren und/oder Glasformen, insbesondere mittels Spritzgießen, Gießformen und/oder Extrudieren, verbunden, insbesondere fest und/oder unlösbar verbunden, vorzugsweise stoffschlüssig verbunden, ist.
- Vorzugsweise ist der Adapter 4 zumindest im Wesentlichen rotationssymmetrisch ausgebildet.

Der Spritzenzylinder 3 ist vorzugsweise zumindest im Wesentlichen rotationssymmetrisch ausgebildet.

Besonders bevorzugt sind der Adapter 4 und der Spritzenzylinder 3 koaxial zueinander angeordnet, insbesondere bezogen auf die Längsachse, insbesondere Rotationsachse, des Adapters 4 und die Längsachse, insbesondere Rotationsachse, des Spritzenzylinders 3.

Insbesondere weisen der Adapter 4 und der Spritzenzylinder 3 eine gemeinsame Längsachse, insbesondere Rotationsachse, auf.

Weiterhin weist der Spritzenzylinder 3 einen zumindest im Wesentlichen kreisrunden Querschnitt auf, wobei der Spritzenzylinder 3 einen zumindest im Wesentlichen konstanten Durchmesser und/oder eine zumindest im Wesentlichen konstante Querschnittsfläche aufweist, insbesondere entlang der Längsachse, insbesondere Rotationsachse, des Spritzenzylinders 3.

Der Spritzenkörper 2 kann zumindest im Wesentlichen rotationssymmetrisch ausgebildet sind.

Erfindungsgemäß weist der Adapter 4 einen vorzugsweise zumindest im Wesentlichen kreisrunden Querschnitt auf, insbesondere wobei der erste Abschnitt des Adapters 4 eine in Auslassrichtung AR und/oder in Richtung der Auslassöffnung 4a des Adapters 4 abnehmende und/oder sich verringernde Querschnittsfläche aufweist.

Was das Verhältnis einer Länge L des Spritzenzylinders 3 zu der I des Adapters 4 anbelangt, so verhält es sich erfindungsgemäß vorzugsweise wie folgt:
- Insbesondere beträgt das Verhältnis der Länge L des Spritzenzylinders 3 zu der Länge I des Adapters 4 mindestens 1,5, insbesondere mindestens 2, vorzugsweise mindestens 2,5, bevorzugt mindestens 3.
- Vorzugsweise beträgt das Verhältnis der Länge L des Spritzenzylinders 3 zu der Länge I des Adapters 4 höchstens 20, insbesondere höchstens 15, vorzugsweise höchstens 10, bevorzugt höchstens 5.
- Besonders bevorzugt liegt das Verhältnis der Länge L des Spritzenzylinders 3 zu der Länge I des Adapters 4 in einem Bereich von 1,5 bis 20, insbesondere in einem Bereich von 2 bis 15, vorzugsweise in einem Bereich von 2,5 bis 10, bevorzugt in einem Bereich von 3 bis 5.

Es hat sich als zweckmäßig erwiesen, wenn die Kolbenspritze 1, insbesondere der Spritzenzylinder 3, ein Volumen, insbesondere ein Aufnahmevolumen für die oxybutyninhaltige Zusammensetzung Z, im Bereich von 1 ml bis 100 ml, insbesondere im Bereich von 2 ml bis 50 ml, vorzugsweise im Bereich von 5 ml bis 25 ml, bevorzugt im Bereich von 4 ml bis 25 ml, besonders bevorzugt im Bereich von 6 ml bis 20 ml, weiter bevorzugt im Bereich von 8 ml bis 15 ml, nochmals weiter bevorzugt im Bereich von 9 ml bis 12 ml, ganz besonders bevorzugt von etwa 10 ml, aufweist.

Es ist zudem oder alternativ bevorzugt, wenn die Kolbenspritze 1, insbesondere der Spritzenzylinder 3, die oxybutyninhaltige Zusammensetzung Z in einer Menge im Bereich von 1 ml bis 100 ml, insbesondere im Bereich von 2 ml bis 50 ml, vorzugsweise im Bereich von 5 ml bis 25 ml, bevorzugt im Bereich von 4 ml bis 25 ml, besonders bevorzugt im Bereich von 6 ml bis 20 ml, weiter bevorzugt im Bereich von 8 ml bis 15 ml, nochmals weiter bevorzugt im Bereich von 9 ml bis 12 ml, ganz besonders bevorzugt von etwa 10 ml, aufweist.

Erfindungsgemäß sind der Spritzenzylinder 3 und der Adapter 4 aus identischen Materialien gebildet.

Insbesondere bestehen der Spritzenzylinder 3 und der Adapter 4 aus demselben Material.

Weiterhin ist vorzugsweise vorgesehen, dass der Spritzenkörper 2, vorzugsweise der Spritzenzylinder 3 und der Adapter 4, aus einem Kunststoffmaterial oder Glasmaterial, vorzugsweise Kunststoffmaterial, gebildet ist bzw. sind, insbesondere wobei der Spritzenkörper 2, vorzugsweise der Spritzenzylinder 3 und der Adapter 4, aus einem Cycloolefin-(Co-)Polymer (COP bzw. COC) gebildet ist bzw. sind.

Es ist bevorzugt, wenn der Spritzenkolben 5 aus einem Kunststoffmaterial oder Glasmaterial, vorzugsweise Kunststoffmaterial, gebildet ist.

Insbesondere ist der Spritzenkolben 5 aus Polypropylen oder aus einem Cycloolefin-(Co-)Polymer (COP bzw. COC), insbesondere aus Polypropylen, gebildet ist.

Gemäß einer besonders bevorzugten Ausführungsform ist der Spritzenkörper 2, vorzugsweise vollständig, aus Polypropylen gebildet oder weist ein Polypropylen auf.

Besonders bevorzugt hat es sich in diesem Zusammenhang erwiesen, dass der Spritzenkolben 5, vorzugsweise vollständig, aus Polypropylen gebildet ist oder ein Polypropylen aufweist.

Ganz besonders bevorzugt ist bzw. sind der Spritzenzylinder 3 und der Adapter 4 und/oder der Spritzenkolben 5, vorzugsweise jeweils vollständig, aus einem Polypropylen gebildet oder weist bzw. weisen ein Polypropylen auf. Gerade die Ausbildung des Spritzenkörpers 2 und/oder des Spritzenzylinders 3 und/oder des Adapters 4 aus Polypropylen hat sich als kostengünstig und effizient erwiesen.

Was eine weitere, bevorzugte Ausführungsform der erfindungsgemäßen Kolbenspritze 1 anbelangt, weist der Spritzenkörper 2, insbesondere der Spritzenzylinder 3, eine Aufnahme- und/oder Befüllöffnung 5a auf, insbesondere zum Befüllen des Spritzenkörpers 2, insbesondere des Spritzenzylinders 3, mit der oxybutyninhaltigen Zusammensetzung Z und/oder insbesondere zur Aufnahme des Spritzenkolbens 5 mit dem Kolbenstopfen 6 und/oder insbesondere wobei die Aufnahme- und/oder Befüllöffnung 5a mit dem Spritzenkolben 5, insbesondere mit dem Kolbenstopfen 6, verschließbar ist und/oder verschließbar ausgebildet ist und/oder verschlossen ist.

Insbesondere ist der Kolbenstopfen 6 aus einem Halogenbutylkautschuk, insbesondere aus einem Chlorbutylkautschuk oder aus einem Brombutylkautschuk, vorzugsweise aus einem Brombutylkautschuk, gebildet.

Es hat sich zudem als zweckmäßig und besonders bevorzugt erwiesen, wenn der Kolbenstopfen 6 lösbar mit dem Spritzenkolben 5 verbindbar und/oder verbunden ist, insbesondere mittels einer Schraubverbindung, insbesondere wobei der Kolbenstopfen 6 eine Gewindevertiefung 17 zum Zusammenwirken mit einem komplementären Schraubabschnitt 18 des Spritzenkolbens 5 aufweist.

Erfindungsgemäß kann die in der Kolbenspritze 1 enthaltene oxybutyninhaltige Zusammensetzung Z einschließlich der Kolbenspritze 1 und das gegebenenfalls vorhandene Verschlusselement 13 dampfsterilisiert, insbesondere wasserdampfsterilisiert, vorliegen.

Im Hinblick darauf wird die Dampfsterilisation unter Overkill-Bedingungen und/oder bei etwa 121 °C und/oder für einen Zeitraum von mindestens 15 min, insbesondere von mindestens 20 min, vorzugsweise von etwa 20 min, durchgeführt, insbesondere wobei die Dampfsterilisation gemäß Pharmacopoea Europaea (Ph. Eur.), Kapitel 5.1.1 (07/2017:50101) durchgeführt ist, insbesondere unter Overkill-Bedingungen und/oder bei etwa 121 °C und/oder für einen Zeitraum von mindestens 15 min, insbesondere von mindestens 20 min, vorzugsweise von etwa 20 min.

Insbesondere ist dabei vorgesehen, dass die mit der nach Dampfsterilisation erhaltene sterile oxybutyninhaltigen Zusammensetzung Z befüllte sterile Kolbenspritze 1 und/oder die nach Dampfsterilisation erhaltene oxybutyninhaltige Zusammensetzung Z einen SAL-Wert (*Sterility Assurance Level*) von mindestens 10⁻⁵, insbesondere mindestens 10⁻⁶, vorzugsweise mindestens 10⁻⁷, aufweist.

Die erfindungsgemäße Kolbenspritze 1 ist erfindungsgemäß insbesondere zur Instillation und/oder zur vorzugsweise topischen Applikation der oxybutyninhaltigen Zusammensetzung Z in den Urogenitalbereich, insbesondere in die Harnblase ausgebildet und/oder vorgesehen.

Was insbesondere die oxybutyninhaltige Zusammensetzung Z anbelangt, so haben sich erfindungsgemäß folgende Sachverhalte als besonders zweckmäßig bzw. vorteilhaft erwiesen:
- Insbesondere enthält die oxybutyninhaltige Zusammensetzung Z das Oxybutynin in Form des Hydrochlorids.
- Vorzugsweise enthält die oxybutyninhaltige Zusammensetzung Z Oxybutyninhydrochlorid (Oxybutynin-HCl).
- Besonders enthält die oxybutyninhaltige Zusammensetzung Z das Oxybutynin in Form des Hydrochlorids und/oder in Form von Oxybutyninhydrochlorid (Oxybutynin-HCl).

Erfindungsgemäß enthält die oxybutyninhaltige Zusammensetzung Z das Oxybutynin, insbesondere Oxybutyninhydrochlorid (Oxybutynin-HCl), vorzugsweise in einer Konzentration von (1 ± 0,5) mg/ml, insbesondere in einer Konzentration von (1 ± 0,4) mg/ml, vorzugsweise in einer Konzentration von (1 ± 0,3) mg/ml, bevorzugt in einer Konzentration von (1 ± 0,2) mg/ml, besonders bevorzugt in einer Konzentration von (1 ± 0,1) mg/ml, ganz besonders bevorzugt in einer Konzentration von etwa 1 mg/ml.

Insbesondere enthält die oxybutyninhaltige Zusammensetzung Z Natriumchlorid, insbesondere wobei die oxybutyninhaltige Zusammensetzung Z Natriumchlorid, berechnet als Natrium, in einer Konzentration von (3,5 ± 0,5) mg/ml, insbesondere in einer Konzentration von (3,5 ± 0,4) mg/ml, vorzugsweise in einer Konzentration von (3,5 ± 0,3) mg/ml, bevorzugt in einer Konzentration von (3,5 ± 0,2) mg/ml, besonders bevorzugt in einer Konzentration von (3,5 ± 0,1) mg/ml, ganz besonders bevorzugt in einer Konzentration von etwa 3,5 mg/ml, enthält.

Es hat sich zudem als zweckmäßig erwiesen, wenn die oxybutyninhaltige Zusammensetzung Z auf einen pH-Wert in einem Bereich von 2,8 bis 5,2, insbesondere in einem Bereich von 3 bis 5, vorzugsweise 3,2 bis 4,8, bevorzugt 3,5 bis 4,5, besonders bevorzugt 3,5 bis 4,2, ganz besonders bevorzugt 3,8 bis 4,2, eingestellt ist, insbesondere wobei die oxybutyninhaltige Zusammensetzung Z einen pH-Wert in einem Bereich von 2,8 bis 5,2, insbesondere in einem Bereich von 3 bis 5, vorzugsweise 3,2 bis 4,8, bevorzugt 3,5 bis 4,5, besonders bevorzugt 3,5 bis 4,2, ganz besonders bevorzugt 3,8 bis 4,2, aufweist.

Die oxybutyninhaltige Zusammensetzung Z kann das Oxybutynin in Form des Hydrochlorids enthalten, wobei die oxybutyninhaltige Zusammensetzung Z als wässrige Zusammensetzung ausgebildet ist und die oxybutyninhaltige Zusammensetzung Z in Kombination und vorzugsweise jeweils in wirksamen, insbesondere pharmazeutisch wirksamen Mengen,
- Oxybutyninhydrochlorid (Oxybutynin-HCl) in einer Konzentration von (1 ± 0,5) mg/ml und/oder in einer pharmazeutisch wirksamen Menge, und
- Natriumchlorid, berechnet als Natrium, in einer Konzentration von (3,5 ± 0,5) mg/ml, insbesondere in einer pharmazeutisch nicht wirksamen Menge,
enthält,
wobei die oxybutyninhaltige Zusammensetzung Z auf einen pH-Wert in einem Bereich von 2,8 bis 5,2, insbesondere in einem Bereich von 3 bis 5, vorzugsweise 3,2 bis 4,8, bevorzugt 3,5 bis 4,5, besonders bevorzugt 3,5 bis 4,2, ganz besonders bevorzugt 3,8 bis 4,2, eingestellt ist, und/oder insbesondere wobei die oxybutyninhaltige Zusammensetzung Z einen pH-Wert in einem Bereich von 2,8 bis 5,2, insbesondere in einem Bereich von 3 bis 5, vorzugsweise 3,2 bis 4,8, bevorzugt 3,5 bis 4,5, besonders bevorzugt 3,5 bis 4,2, ganz besonders bevorzugt 3,8 bis 4,2, aufweist.

Vorzugsweise ist die oxybutyninhaltige Zusammensetzung Z als wässrige Zusammensetzung ausgebildet und/oder zur Verwendung bei der prophylaktischen und/oder therapeutischen Behandlung von neurogenen Blasenfunktionsstörungen, insbesondere neurogenen Blasenentleerungsstörungen, vorzugsweise von mit einer Detrusorhyperaktivität und/oder einer Detrusor-Sphinkter-Dyssynergie einhergehenden neurogenen Blasenfunktionsstörungen, vorgesehen.

Dabei gehen die neurogenen Blasenfunktionsstörungen vorzugsweise mit einer Detrusorüberaktivität und/oder Detrusor-Sphinkter-Dyssynergie, insbesondere mit überaktivem Detrusor, einher und/oder sind hierdurch gekennzeichnet, insbesondere wobei die neurogenen Blasenfunktionsstörungen mit einer Rückenmarksverletzung, insbesondere einer Querschnittlähmung, Spina bifida, Diabetes, Multiple Sklerose, Schlaganfall oder Morbus Parkinson im Zusammenhang stehen oder hierdurch verursacht sind.

Darüber hinaus ist vorliegend beschrieben zudem ein Spritzenkörper 2, insbesondere zur Verwendung als Bestandteil und/oder zur Ausbildung einer Kolbenspritze 1, vorzugsweise Einweg-Kolbenspritze, insbesondere wie zuvor definiert,
wobei der Spritzenkörper 2 einen Spritzenzylinder 3, insbesondere zur Aufnahme einer oxybutyninhaltigen Zusammensetzung Z, und einen Adapter 4, insbesondere zum Anschluss einer Instillationsvorrichtung, aufweist, wobei der Spritzenkörper 2 einstückig (einteilig) ausgebildet ist und/oder wobei der Adapter 4 mit dem Spritzenzylinder 3 verbunden, insbesondere fest und/oder unlösbar verbunden, vorzugsweise stoffschlüssig verbunden, ist,
wobei der Adapter 4 mindestens einen, vorzugsweise genau einen, ersten Abschnitt A₁ mit einem sich in Auslassrichtung vorzugsweise kontinuierlich verjüngenden und/oder verringernden Außenumfang und/oder Durchmesser, insbesondere Außendurchmesser, aufweist, und
wobei der Adapter 4 mindestens einen, vorzugsweise genau einen, sich in Auslassrichtung vorzugsweise unmittelbar an den ersten Abschnitt A₁ anschließenden zweiten, zylindrischen Abschnitt A₂ mit einem gegenüber dem ersten Abschnitt A₁ verringerten Außenumfang und/oder Durchmesser, insbesondere Außendurchmesser, aufweist.

Für weitergehende Einzelheiten und Ausführungen bezüglich des Spritzenkörpers 2 der vorliegenden Erfindung kann auf die voranstehenden Ausführungen im Zusammenhang mit der erfindungsgemäßen Kolbenspritze 1 verwiesen werden, welche für den Spritzenkörper 2 entsprechend gelten.

Vorliegend beschrieben ist auch eine Verpackungseinheit, enthaltend mindestens eine Verpackung und mindestens eine Kolbenspritze 1, insbesondere Einweg-Kolbenspritze, wie vorangehend definiert, wobei die Kolbenspritze 1 in die Verpackung eingebracht ist bzw. in der Verpackung vorliegt.

Diesbezügliche Ausgestaltungen der Verpackungseinheit kann zudem auf die Ausführungen zu den weiteren Aspekten verwiesen werden, welche entsprechend gelten.

Wiederum weiterer Gegenstand der vorliegenden Erfindung - gemäß einem **weiteren** Aspekt der vorliegenden Erfindung - ist zudem das erfindungsgemäße Kit, insbesondere Applikations- und/oder Instillationssystem, umfassend (i) mindestens eine in einer Kolbenspritze 1, insbesondere Einweg-Kolbenspritze, wie zuvor definiert, vorliegende oxybutyninhaltige Zusammensetzung Z, insbesondere wie vorliegend definiert; (ii) (a) mindestens eine an die Kolbenspritze 1, insbesondere an den Adapter 4 der Kolbenspritze 1 anschließbare Applikations- und/oder Instillationsvorrichtung, insbesondere in Form eines Instillationsschlauches oder dergleichen, vorzugsweise in Form eines (Harnblasen-)Katheters; gegebenenfalls (iii) ein Adapter-Gegenstück zum Anschließen der Applikations- und/oder Instillationsvorrichtung an die Kolbenspritze 1, insbesondere an den Adapter 4 der Kolbenspritze 1; und gegebenenfalls (iv) mindestens eine Applikations- und/oder Instillationsanleitung.

Diesbezügliche Ausgestaltungen des erfindungsgemäßen Kits kann zudem auf die Ausführungen zu den weiteren erfindungsgemäßen Aspekten verwiesen werden, welche entsprechend gelten.

Wiederum weiterer Gegenstand der vorliegenden Erfindung - gemäß einem **weiteren** Aspekt der vorliegenden Erfindung - ist zudem die erfindungsgemäße Verwendung einer Kolbenspritze, insbesondere Einweg-Kolbenspritze, in einem Verfahren zur Herstellung einer sterilen oxybutyninhaltigen Zusammensetzung, wie zuvor definiert,
wobei die Kolbenspritze 1 mit der sterilen oxybutyninhaltigen Zusammensetzung Z, insbesondere zur Verwendung bei der prophylaktischen und/oder therapeutischen Behandlung von neurogenen Blasenfunktionsstörungen, insbesondere neurogenen Blasenentleerungsstörungen, vorzugsweise von mit einer Detrusorhyperaktivität und/oder einer Detrusor-Sphinkter-Dyssynergie einhergehenden neurogenen Blasenfunktionsstörungen, befüllt ist und
wobei die in der Kolbenspritze enthaltene oxybutyninhaltige Zusammensetzung Z einschließlich der Kolbenspritze 1 dampfsterilisiert, insbesondere wasserdampfsterilisiert, wird.

Für diesbezügliche Ausgestaltungen der Kolbenspritze 1 im Rahmen der erfindungsgemäßen Verwendung zudem auf die Ausführungen zu den weiteren erfindungsgemäßen Aspekten verwiesen werden, welche entsprechend gelten.

Schließlich ist ein weiterer Gegenstand der vorliegenden Erfindung - gemäß einem **weiteren** Aspekt der vorliegenden Erfindung - zudem die erfindungsgemäße Verwendung einer Dampfsterilisation, insbesondere Wasserdampfsterilisation, in einem Verfahren zur Herstellung einer sterilen oxybutyninhaltigen Zusammensetzung in einer vorzugsweise anwendungsfertigen Kolbenspritze, insbesondere Einweg-Kolbenspritze, und/oder zur Herstellung einer vorzugsweise anwendungsfertigen, sterilen, mit einer oxybutyninhaltigen Zusammensetzung befüllten Kolbenspritze, insbesondere Einweg-Kolbenspritze, wie zuvor definiert, wobei die mit der oxybutyninhaltigen Zusammensetzung Z befüllte Kolbenspritze 1 und/oder die in der Kolbenspritze 1 bereitgestellte oxybutyninhaltige Zusammensetzung Z dampfsterilisiert, insbesondere wasserdampfsterilisiert, wird.

Für weitere diesbezügliche Ausführungen zu der erfindungsgemäßen Verwendung gemäß diesem Aspekt kann auch auf die Ausführungen zu den weiteren erfindungsgemäßen Aspekten verwiesen werden, welche vorliegend entsprechend gelten.

Weitere Ausgestaltungen, Abwandlungen und Variationen sowie Vorteile der vorliegenden Erfindung sind für den Fachmann beim Lesen der Beschreibung ohne weiteres erkennbar und realisierbar, ohne dass er dabei den Rahmen der vorliegenden Erfindung verlässt.

Die folgenden Ausführungsbeispiele dienen lediglich der Veranschaulichung der vorliegenden Erfindung, jedoch ohne die vorliegende Erfindung hierauf zu beschränken.

### AUSFÜHRUNGSBEISPIELE:

Die nachfolgend beschriebenen Ausführungen zeigen die verbesserten Eigenschaften der erfindungsgemäßen Einweg-Kolbenspritze mit der einteiligen Ausbildung des Spritzenkörpers aus Zylinder und Adapter, welche mit einer sterilen oxybutyninhaltigen Zusammensetzung befüllt ist.

Die nachfolgend beschriebenen Untersuchungen wurden dabei zur Darlegung der verbesserten Stabilität der Zusammensetzung bei der Sterilisation mit der Verringerung von Abbauprodukten von Oxybutyninhydrochlorid einerseits, aber auch im Hinblick auf Untersuchungen zur verbesserten Handhabbarkeit, erhöhten Dichtigkeit sowie erhöhten Kompatibilität andererseits entsprechend durchgeführt.

### a) Sterilisationsprüfung

Dabei werden gemäß einer erfindungsgemäßen Ausführungsform erfindungsgemäße Kolbenspritzen auf Basis von Cycloolefin-(Co-)Polymeren (COP bzw. COC) und Kolbenstopfen bzw. Verschlusskappen bevorzugt auf Basis von Halogenbutylkautschuk, insbesondere Chlorbutylkautschuk oder Brombutylkautschuk, vorzugsweise Brombutylkautschuk, eingesetzt.

Gemäß einer alternativen Ausführungsform werden erfindungsgemäße Kolbenspritzen auf Basis von Polypropylen und Kolbenstopfen bzw. Verschlusskappen bevorzugt auf Basis von Halogenbutylkautschuk, insbesondere Chlorbutylkautschuk oder Brombutylkautschuk, vorzugsweise Brombutylkautschuk, eingesetzt. Hiermit lassen sich zumindest vergleichbare, vorzugsweise sogar verbesserte, Ergebnisse im Hinblick auf die Sterilisationsprüfung erzielen lassen.

Insbesondere verhält es sich bei einer terminalen Dampfsterilisation einer Zusammensetzung zur Instillation in die Blase, welche den Wirkstoff Oxybutyninhydrochlorid in einer Konzentration von 1 mg/ml bei einem pH-Wert der Zusammensetzung von 4,0, enthält und welche entweder in COP- bzw. COC-Kolbenspritzen oder alternativ in Polypropylenkolbenspritzen jeweils mit Kolbenstopfen aus einem üblichen Gummimaterial vorgefüllt ist, derart, dass nach Durchführung einer Dampfsterilisation der Gehalt an Oxybutynin auf 85 bis 90 % des zuvor vorliegenden Wirkstoffgehalts absinkt. Dabei entsteht bei der Dampfsterilisierung in den Kolbenspritzen mit Kolbenstopfen aus herkömmlichem Gummimaterial das Oxybutynin-Abbauprodukt Phenylcyclohexylglycolsäure, und zwar in solchen Mengen, welche um einen Faktor 2 bis 3 oberhalb der Spezifikationsgrenze liegen; zudem resultieren nach dem Stand der Technik weitere Verunreinigungen in Mengen von teilweise über 0,5 %.

Ohne sich auf diese Theorie beschränken zu wollen, liegt - im Gegensatz zu den erfindungsgemäß verwendeten Materialien - für andere Materialien mitunter eine nur geringe Säurebeständigkeit vor, wobei die bei der Dampfsterilisierung vorliegenden hohen Temperaturen zudem mögliche Reaktionen zwischen der Zusammensetzung und dem Spritzenmaterial begünstigen, was auch zur Ausbildung von den weiteren Verunreinigungen führen kann. Dabei verhält es sich für das Abbauprodukt von Oxybutyninhydrochlorid in Form von Phenylcyclohexylglycolsäure insbesondere derart, dass dieses grundsätzlich durch zwei unterschiedliche Reaktionen entstehen kann, und zwar einerseits aus einer Verseifung in alkalischer Lösung oder andererseits aus einer Hydrolyse in saurer Lösung. Da der pH-Wert der Zusammensetzung im sauren Bereich liegt, kann - ohne sich auf diese Theorie beschränken zu wollen - davon ausgegangen werden, dass das Abbauprodukt vorrangig aus einer Hydrolyse von Oxybutyninhydrochlorid hervorgeht, wobei diese Reaktion durch die bei der Sterilisation vorliegenden hohen Temperaturen nochmals verstärkt wird. Durch die Hydrolyse des Wirkstoffs resultiert sowohl eine Verringerung des Wirkstoffgehalts als auch die Bildung von Phenylcyclohexylglycolsäure bei vorliegender Dampfsterilisation. Zudem wird - im Gegensatz zu der erfindungsgemäß bevorzugten Vorgehensweise - im Stand der Technik die Bildung von Phenylcyclohexylglycolsäure infolge des für den Kolbenstopfen im Allgemeinen verwendeten Gummimaterials weiterführend verstärkt bzw. beschleunigt. Ohne sich auf diese Theorie beschränken zu wollen, verhält es sich im Allgemeinen derart, dass Gummimaterialien, wie sie im Allgemeinen für Kolbenstopfen eingesetzt werden können, mitunter einen hohen Anteil an ungesättigten C-C-Doppelbindungen aufweisen können, was eine erhöhte Reaktivität des Materials zur Folge hat. Zudem verhält es sich - ohne sich auf diese Theorie beschränken zu wollen - für Phenylcyclohexylglycolsäure derart, dass diese durch die freie Säuregruppe reaktiver ist als die Wirksubstanz, so dass bei der Sterilisation entstehende Phenylcyclohexylglycolsäure zumindest teilweise mit dem Gummimaterial reagiert und so dem Reaktionsgleichgewicht entzogen wird, was den Abbau der Wirksubstanz weiter bevorzugt.

Im Gegensatz hierzu wird im Rahmen der erfindungsgemäß bevorzugten Ausführungsform unter Verwendung von erfindungsgemäßen Kolbenspritzen insbesondere auf Basis von Cycloolefin-(Co-)Polymeren (COP bzw. COC) (oder alternativ: Polypropylen) und Kolbenstopfen bzw. Verschlusskappen bevorzugt auf Basis von Halogenbutylkautschuk, insbesondere Chlorbutylkautschuk oder Brombutylkautschuk, vorzugsweise Brombutylkautschuk, und einer entsprechenden oxybutyninhaltigen Zusammensetzung eine deutlich verbesserte Stabilität der Zusammensetzung mit einem geringerem Abbau der Wirksubstanz und somit einer verringerten Bildung von Abbauprodukten, insbesondere Phenylcyclohexylglycolsäure, unter definierter Dampfsterilisation beobachtet. Das erfindungsgemäß für die Kolbenspritze insbesondere eingesetzte Cycloolefin-(Co-) Polymer (COP bzw. COC) weist dabei im Gegensatz zu anderen Materialien eine hohe Stabilität auch gegenüber sauren Zusammensetzungen auf (wie auch Polypropylen unter entsprechenden Sterilisationsbedingungen; vgl. hierzu auch DE 10 2021 112 033.0 und parallele PCT/EP 2022/054205).

Zudem verhält es sich in Bezug auf den Kolbenstopfen bzw. der Verschlusskappe derart, dass der erfindungsgemäß hierfür bevorzugt eingesetzte Halogenbutylkautschuk, wie zuvor angeführt, eine wesentlich geringere Anzahl an C-C-Doppelbindungen aufweist und somit weniger reaktiv ist als im Allgemeinen eingesetzte Gummimaterialien. Zudem weist Halogenbutylkautschuk eine verbesserte Hitzestabilität und verbesserte Alterungseigenschaften und eine geringe Gaspermeabilität auf.

Auf dieser Basis kann im Rahmen der vorliegenden Erfindung bei der Dampfsterilisation eine entsprechend sterile oxybutyninhaltige Zusammensetzung mit verbesserten Eigenschaften erhalten werden, und zwar sowohl im Hinblick auf die Gewährleistung einer hohen Sterilität als auch im Hinblick auf die Gewährleistung eines hohen bzw. unveränderten Wirkstoffgehalts, bedingt durch eine deutliche Verringerung der Bildung entsprechender Abbauprodukte.

### b) Handhabbarkeits- und Dichtigkeitsprüfung

Überdies wurde unter Verwendung der erfindungsgemäßen Kolbenspritzen sowie einer Vielzahl auf dem Markt befindlicher Katheter-Systeme ein Aufsetzen sowie auch ein Instillieren simuliert. Anhand der unterschiedlichen Druckaufwände (jeweilige Messung durch Tester) wurde sowohl das Aufbringen auf den Urinbeutelanschluss wie auch die Aufsitzhaftung bei Instillation geprüft. Der Druckwiderstand bei Instillation wurde mittels Abknicken des Katheters erzeugt. Der Anschluss durfte sich nicht bei spürbarem Druck ablösen oder Flüssigkeit austreten.

Es wurde insgesamt eine erfindungsgemäße Kolbenspritze, welche einen stoffschlüssig verbundenen Adapter aufweist (d. h. erfindungsgemäße einstückige Ausbildung) im Vergleich zu sieben unterschiedlichen, kommerziell verfügbaren Adapterformen mit jeweils zugeordneten Kolbenspritzen des Standes der Technik untersucht, wobei seitens der Probanden folgende Bewertungen vorgenommen worden sind:

| | |
|---|---|
| ++: | einfach, dicht aufsetzbar |
| +: | schwergängig, aber dicht aufsetzbar |
| ∘: | nur mit Überlappung dicht aufsetzbar |
| -: | undicht, locker. |

Im Rahmen der Erprobung wurden die nachfolgenden kommerziell verfügbaren Katheter-Systeme im Zusammenhang mit den vorgenannten Adapterversionen des Standes der Technik verwendet:

| **Marke** | **ArtDK/ISK** | **Material** | **Abmessungen Katheteranschluss max.** |
|---|---|---|---|
| Uromed | DK14Ch. | Silicon | 9,18 mm |
| Well Lead | DK14Ch. | Silicon | 8,30 mm |
| Coloplast | DK16Ch. | Silicon | 9,70 mm |
| Rüsch/Teleflex | DK14Ch. | Beschichteter Latex | 9,06 mm |
| Rüsch/SymohaCath | DK16Ch. | Beschichteter Latex | 8,65 mm |
| Medfein | DK14Ch. | Silicon | 9,26 mm |
| Teleflex SafetyCath M | ISK14Ch. | PVC | 7,50 mm |
| Teleflex SafetyCath P | ISK12Ch. | PVC | 7,69 mm |
| Manfred Sauer / IQ Cath | ISK12Ch. | k.A. (PVC) | 9,14 mm |
| Coloplast / SpeedyCath Flex | ISK14CH. | PVC/Nelaton | 8,10 mm |
| Hollister Infyna Chic | ISK12Ch. | PVC/Nelaton | 7,35 mm |
| Wellspect Healthcare Lofric Origa | ISK16Ch. | Nelaton | 8,40 mm |

Die erfindungsgemäßen, einstückig ausgebildeten Kolbenspritzen (d. h. Spritze mit einstückig verbundenem bzw. integriertem Adapter) wurden mit den vorgenannten, aus dem Stand der Technik bekannten Kolbenspritzen inklusive kommerziellen Adapterversionen verglichen, wobei ein Aufsetzen sowie auch ein Instillieren simuliert worden ist,

Anhand der unterschiedlichen Druckaufwände (Messung durch Tester) wurde sowohl das Aufbringen auf den Urinbeutelanschluss wie auch die Aufsitzhaftung bei Instillation geprüft. Der Druckwiderstand bei Instillation wurde mittels Abknicken des Katheter erzeugt. Der Anschluss durfte sich nicht bei spürbarem Druck ablösen oder Flüssigkeit austreten.

Im Anschluss daran wurden die verwendeten Adapter bzw. Kolbenspritzen für jedes Katheter-System bewertet und die Ergebnisse dann zusammengetragen bzw. verglichen.

Im Rahmen der praktischen Erprobung konnte dabei gezeigt werden, dass nur eine mit dem erfindungsgemäß vorgesehenen, stoffschlüssig verbundenen und erfindungsgemäß ausgebildeten Adapter ausgerüstete Kolbenspritze 1 (vgl. Figs. 1 bis 5) für sämtliche der obigen aufgelisteten Katheter-Systeme jeweils die Maximalbewertung (d. h. Bewertung: ++) erzielt hat und sich insofern gegenüber den zu vergleichenden Kolbenspritzen bzw. Adaptern des Standes der Technik hervortat. Für keine der aus dem Stand der Technik bekannten Adapterversionen konnte eine Maximalbewertung für alle unterschiedlichen Katheter-Systeme erzielt werden; die Bewertungen für die Systeme des Standes der Technik lagen jeweils nur bei den vorgenannten Bewertungen "+" bzw. "∘" bzw. "-".

### c) Zusammenfassung

Insgesamt ist somit festzustellen, dass auf Basis des erfindungsgemäßen Verfahrens unter Verwendung einer speziellen oxybutyninhaltigen Zusammensetzung und unter Verwendung spezieller Kolbenspritzen nach der Erfindung mit diesbezüglich speziellen Komponenten eine effiziente und zugleich schonende Dampfsterilisation der zugrundeliegenden Zusammensetzung durchgeführt werden kann, wobei der Abbau der Wirksubstanz verringert wird, so dass auch diesbezüglich entsprechend stabile Zusammensetzungen bereitgestellt werden. Überdies konnte im Rahmen der praktischen Erprobung dargelegt werden, dass durch eine mit dem erfindungsgemäß vorgesehenen Adapter ausgerüstete Kolbenspritze eine zuverlässig hohe Dichtwirkung bei gleichzeitig einfacher Handhabung und hoher Kompatibilität sichergestellt ist, und zwar für eine Vielzahl möglicher Katheter-Systeme.

### Bezugszeichenliste:

- 1: Kolbenspritze
- 2: Spritzenkörper
- 3: Spritzenzylinder
- 4: Adapter
- 4a: Auslassöffnung
- 4b: Einlassöffnung
- 5: Spritzenkolben
- 5a: Befüllöffnung
- 6: Kolbenstopfen
- 7: Stufe
- 8: Übergang
- 9: Durchlasskanal
- 10: Vorsprung
- 11: Rundung
- 12: Übergang
- 13: Verschlusselement
- 14: Grundkörper
- 15: Griffabschnitt
- 16: Dichtabschnitt
- 17: Gewindevertiefung
- 18: Schraubenabschnitt

- A₁: erster Abschnitt
- A₂: zweiter Abschnitt
- A₃: dritter Abschnitt
- d: Durchlassdurchmesser
- dₐᵤₛ: Auslassdurchmesser
- Dₘᵢₙ₁: minimaler Durchmesser des ersten Abschnitts
- Dₘₐₓ₁: maximaler Durchmesser des ersten Abschnitts
- D₂: Durchmesser des zweiten Abschnitts
- Dₘᵢₙ₃: minimaler Durchmesser des dritten Abschnitts
- Dₘₐₓ₃: maximaler Durchmesser des dritten Abschnitts
- l: Länge des Adapters
- l₁: Länge des ersten Abschnitts des Adapters
- l₂: Länge des zweiten Abschnitts des Adapters
- l₃: Länge des dritten Abschnitts des Adapters
- L: Länge des Spritzenzylinders
- Z: Oxybutyninhaltige Zusammensetzung
- AR: Auslassrichtung

## Patentansprüche

1. Kolbenspritze (1), insbesondere Einweg-Kolbenspritze,
wobei die Kolbenspritze (1)
- einen Spritzenkörper (2), welcher einen Spritzenzylinder (3) zur Aufnahme einer vorzugsweise oxybutyninhaltigen Zusammensetzung (Z), und einen Adapter (4) zum Anschluss an eine Instillationsvorrichtung aufweist, wobei der Spritzenkörper (2) einstückig ausgebildet ist und/oder wobei der Adapter (4) mit dem Spritzenzylinder (3) fest und/oder unlösbar verbunden ist, und
- einen Spritzenkolben (5) mit einem Kolbenstopfen (6)
aufweist,
wobei der Adapter (4) mindestens einen ersten Abschnitt (A₁) mit einem sich in Auslassrichtung (AR) kontinuierlich verjüngenden Außenumfang und/oder Außendurchmesser aufweist, und
wobei der Adapter (4) mindestens einen sich in Auslassrichtung (AR) unmittelbar an den ersten Abschnitt (A₁) anschließenden zweiten, zylindrischen Abschnitt (A₂) mit einem gegenüber dem ersten Abschnitt (A₁) verringerten Außenumfang aufweist,
wobei sich der zweite Abschnitt (A₂) hin zu einer Auslassöffnung (4a) des Adapters (4) erstreckt und die Auslassöffnung (4a) am freien Ende des zweiten Abschnitts (A₂) ausgebildet ist,
wobei sich der erste Abschnitt (A₁) ausgehend von einem maximalen Außendurchmesser (Dₘₐₓ₁) des ersten Abschnitts (A₁) in Auslassrichtung (AR) zu einem minimalen Außendurchmesser (Dₘᵢₙ₁) des ersten Abschnitts (A₁) verjüngt und das Verhältnis des maximalen Außendurchmessers (Dₘₐₓ₁) des ersten Abschnitts (A₁) zu dem minimalen Außendurchmesser (Dₘᵢₙ₁) des ersten Abschnitts (A₁) in einem Bereich von 1,1 bis 1,6 liegt,
**dadurch gekennzeichnet, dass** am Übergang vom ersten Abschnitt (A₁) zum zweiten Abschnitt (A₂) genau eine Stufe (7) ausgebildet ist,
wobei wenigstens ein dem ersten Abschnitt (A₁) unmittelbar vorgelagerter dritter Abschnitt (A₃) des Adapters (4) vorgesehen ist,
wobei der dritte Abschnitt (A₃) einen sich in Auslassrichtung (AR) kontinuierlich verjüngenden Außenumfang und/oder Außendurchmesser aufweist, wobei der dritte Abschnitt (A₃) stufenfrei in den ersten Abschnitt (A₁) übergeht und
wobei das Verhältnis eines maximalen Außendurchmessers (Dₘₐₓ₃) des dritten Abschnitts (A₃) zu einem minimalen Außendurchmesser (Dₘᵢₙ₃) des dritten Abschnitts (A₃) in einem Bereich von 1,01 bis 1,06 liegt.

2. Kolbenspritze (1) nach Anspruch 1,
wobei der Adapter (4) einen zwischen der Auslassöffnung (4a) des Adapters (4) und der Einlassöffnung (4b) des Adapters (4) befindlichen Hohlraum, insbesondere Durchlasskanal (9), aufweist, insbesondere wobei sich der Hohlraum, insbesondere Durchlasskanal (9), von der Einlassöffnung (4b) des Adapters (4) zu der Auslassöffnung (4a) des Adapters (4) erstreckt und/oder die Einlassöffnung (4b) mit der Auslassöffnung (4a) verbindet.

3. Kolbenspritze (1) nach Anspruch 1 oder 2,
wobei an der Stufe (7), insbesondere an einem an den zweiten Abschnitt (A₂) angrenzenden Bereich der Stufe (7), ein abgerundeter Übergang (8) vorgesehen ist, insbesondere wobei der Übergang (8) einen Radius im Bereich von 0,1 mm bis 0,6 mm, vorzugsweise im Bereich von 0,2 mm bis 0,5 mm, insbesondere im Bereich von 0,3 mm bis 0,4 mm, aufweist.

4. Kolbenspritze (1) nach einem der vorhergehenden Ansprüche,
wobei der erste Abschnitt (A₁) konisch ausgebildet ist; und/oder
wobei das Verhältnis des maximalen Außendurchmessers (Dₘₐₓ₁) des ersten Abschnitts (A₁) zu dem minimalen Außendurchmesser (Dₘᵢₙ₁) des ersten Abschnitts (A₁) in einem Bereich von 1,15 bis 1,5, vorzugsweise in einem Bereich von 1,2 bis 1,45, bevorzugt in einem Bereich von 1,25 bis 1,4, besonders bevorzugt in einem Bereich von 1,3 bis 1,35, liegt; und/oder
wobei das Verhältnis des minimalen Außendurchmessers (Dₘᵢₙ₁) des ersten Abschnitts (A₁) zu dem Außendurchmesser (D₂) des zweiten Abschnitts (A₂) in einem Bereich von 1,1 bis 2, insbesondere in einem Bereich von 1,2 bis 1,9, vorzugsweise in einem Bereich von 1,3 bis 1,8, bevorzugt in einem Bereich von 1,4 bis 1,7, insbesondere in einem Bereich von 1,5 bis 1,6, liegt.

5. Kolbenspritze (1) nach einem der vorangehenden Ansprüche,
wobei sich der zweite Abschnitt (A₂) ausgehend von dem minimalen Außendurchmesser (Dₘᵢₙ₁) des ersten Abschnitts (A₁) hin zu Auslassöffnung (4a) des Adapters (4) erstreckt.

6. Kolbenspritze (1) nach einem der vorangehenden Ansprüche,
wobei der dritte Abschnitt (A₃) und der erste Abschnitt (A₁) an ihrem gemeinsamen Übergang denselben Außendurchmesser aufweisen; und/oder
wobei der dritte Abschnitt (A₃) unmittelbar angrenzend an den Spritzenkörper (2), vorzugsweise an den Spritzenzylinder (3), ausgebildet und/oder angeordnet ist.

7. Kolbenspritze (1) nach Anspruch 6,
wobei das Verhältnis des maximalen Außendurchmessers (Dₘₐₓ₃) des dritten Abschnitts (A₃) zu dem minimalen Außendurchmesser (Dₘᵢₙ₃) des dritten Abschnitts (A₃) in einem Bereich von 1,02 bis 1,05, vorzugsweise in einem Bereich von 1,03 bis 1,045, bevorzugt in einem Bereich von 1,035 bis 1,04, liegt, und/oder
wobei das Verhältnis des maximalen Außendurchmessers (Dₘₐₓ₃) des dritten Abschnitts (A₃) zu dem minimalen Außendurchmesser (Dₘᵢₙ₁) des ersten Abschnitts (A₁) und/oder des Durchmessers an der Stufe des Adapters (4) in einem Bereich von 1,05 bis 1,75, insbesondere in einem Bereich von 1,15 bis 1,65, vorzugsweise in einem Bereich von 1,25 bis 1,55, bevorzugt in einem Bereich von 1,35 bis 1,45, liegt; und/oder
wobei das Verhältnis eines maximalen Außendurchmessers (Dₘₐₓ₃) des dritten Abschnitts (A₃) des Adapters (4) zu dem Außendurchmesser (D₂) des zweiten Abschnitts (A₂) des Adapters (4) in einem Bereich von 1,7 bis 2,5, insbesondere in einem Bereich von 1,8 bis 2,4, vorzugsweise in einem Bereich von 1,9 bis 2,3, bevorzugt in einem Bereich von 2 bis 2,2, liegt.

8. **Kolbenspritze** (1) nach einem der vorangehenden Ansprüche,
wobei das Verhältnis der Länge (l₁) des ersten Abschnitts (A₁) des Adapters (4) zu der Länge (l₂) des zweiten Abschnitts (A₂) des Adapters (4) in einem Bereich von 1,1 bis 1,8, insbesondere in einem Bereich von 1,2 bis 1,7, vorzugsweise in einem Bereich von 1,3 bis 1,6, bevorzugt in einem Bereich von 1,4 bis 1,5, liegt; und/oder
wobei das Verhältnis der Länge (l₁) des ersten Abschnitts (A₁) des Adapters (4) zu der Länge (l₃) des dritten Abschnitts (A₃) des Adapters (4) in einem Bereich von 1,6 bis 2,4, insbesondere in einem Bereich von 1,7 bis 2,3, vorzugsweise in einem Bereich von 1,8 bis 2,2, bevorzugt in einem Bereich von 1,9 bis 2,1 liegt; und/oder
wobei das Verhältnis der Länge (l₂) des zweiten Abschnitts (A₂) des Adapters (4) zu der Länge (l₃) des dritten Abschnitts (A₃) des Adapters (4) in einem Bereich von 1,1 bis 1,8, insbesondere in einem Bereich von 1,2 bis 1,7, vorzugsweise in einem Bereich von 1,3 bis 1,6, bevorzugt in einem Bereich von 1,4 bis 1,5 liegt.

9. Kolbenspritze (1) nach einem der vorangehenden Ansprüche,
wobei der Spritzenkörper (2) mittels Verkleben, Verschweißen, Verschmelzen und/oder Gießen einstückig ausgebildet ist; und/oder
wobei die Verbindung, insbesondere die feste und/oder unlösbare Verbindung, vorzugsweise die stoffschlüssige Verbindung, des Adapters (4) mit dem Spritzenzylinder (3) mittels Verkleben, Verschweißen, Verschmelzen und/oder Gießen erzeugt ist; und/oder
wobei der Adapter (4) mit dem Spritzenzylinder (3) mittels Verkleben, Verschweißen, Verschmelzen und/oder Gießen verbunden, insbesondere fest und/oder unlösbar verbunden, vorzugsweise stoffschlüssig verbunden, ist; und/oder
wobei der Spritzenkörper (2) mittels Spritzgießen, Gießformen, Extrudieren und/oder Glasformen, insbesondere mittels Spritzgießen, Gießformen und/oder Extrudieren, einstückig ausgebildet ist; und/oder
wobei die Verbindung, insbesondere die feste und/oder unlösbare Verbindung, vorzugsweise die stoffschlüssige Verbindung, des Adapters (4) mit dem Spritzenzylinder (3) mittels Spritzgießen, Gießformen, Extrudieren und/oder Glasformen, insbesondere mittels Spritzgießen, Gießformen und/oder Extrudieren, erzeugt ist; und/oder
wobei der Adapter (4) mit dem Spritzenzylinder (3) mittels Spritzgießen, Gießformen, Extrudieren und/oder Glasformen, insbesondere mittels Spritzgießen, Gießformen und/oder Extrudieren, verbunden, insbesondere fest und/oder unlösbar verbunden, vorzugsweise stoffschlüssig verbunden, ist; und/oder
wobei der Adapter (4) zumindest im Wesentlichen rotationssymmetrisch ausgebildet ist.

10. Kolbenspritze (1) nach einem der vorangehenden Ansprüche,
wobei der Spritzenkörper (2), vorzugsweise der Spritzenzylinder (3) und der Adapter (4), aus einem Kunststoffmaterial oder Glasmaterial, vorzugsweise Kunststoffmaterial, gebildet ist bzw. sind; und/oder
wobei der Spritzenkörper (2), vorzugsweise der Spritzenzylinder (3) und der Adapter (4), aus Polypropylen gebildet ist oder ein Polypropylen aufweist; oder
wobei der Spritzenkörper (2), vorzugsweise der Spritzenzylinder (3) und der Adapter (4), aus einem Cycloolefin-(Co-)Polymer (COP bzw. COC) gebildet ist bzw. sind.

11. Kolbenspritze (1) nach einem der vorangehenden Ansprüche,
wobei die in der Kolbenspritze (1) enthaltene oxybutyninhaltige Zusammensetzung (Z) einschließlich der Kolbenspritze (1) und das gegebenenfalls vorhandene Verschlusselement (13) dampfsterilisiert, insbesondere wasserdampfsterilisiert, ist bzw. sind und/oder vorliegt bzw. vorliegen,
insbesondere wobei die Dampfsterilisation unter Overkill-Bedingungen und/oder bei etwa 121 °C und/oder für einen Zeitraum von mindestens 15 min, insbesondere von mindestens 20 min, vorzugsweise von etwa 20 min, durchgeführt ist; und/oder
insbesondere wobei die Dampfsterilisation gemäß Pharmacopoea Europaea (Ph. Eur.), Kapitel 5.1.1 (07/2017:50101) durchgeführt ist, insbesondere unter overkill-Bedingungen und/oder bei etwa 121 °C und/oder für einen Zeitraum von mindestens 15 min, insbesondere von mindestens 20 min, vorzugsweise von etwa 20 min, und/oder
insbesondere wobei die mit der nach Dampfsterilisation erhaltenen sterilen oxybutyninhaltigen Zusammensetzung (Z) befüllte sterile Kolbenspritze (1) und/oder die nach Dampfsterilisation erhaltene oxybutyninhaltige Zusammensetzung (Z) einen SAL-Wert (*Sterility Assurance Level*) von höchstens 10⁻⁵, insbesondere höchstens 10⁻⁶, vorzugsweise höchstens 10⁻⁷, aufweist.

12. Kit, insbesondere Applikations- und/oder Instillationssystem, umfassend (i) mindestens eine Kolbenspritze (1) wie in einem der Ansprüche 1 bis 11 definiert;
(ii)
(a) mindestens eine an die Kolbenspritze (1), insbesondere an den Adapter (4) der Kolbenspritze (1), anschließbare Applikations- und/oder Instillationsvorrichtung, insbesondere in Form eines Instillationsschlauches oder dergleichen, vorzugsweise in Form eines (Harnblasen-)Katheters; gegebenenfalls (iii) ein Adapter-Gegenstück zum Anschließen der Applikations- und/oder Instillationsvorrichtung an die Kolbenspritze (1), insbesondere an den Adapter (4) der Kolbenspritze (1); und gegebenenfalls (iv) mindestens eine Applikations- und/oder Instillationsanleitung.

13. Verwendung einer nach einem der vorangehenden Ansprüche definierten Kolbenspritze (1) in einem Verfahren zur Herstellung einer sterilen oxybutyninhaltigen Zusammensetzung (Z),
wobei die Kolbenspritze (1) mit der sterilen oxybutyninhaltigen Zusammensetzung (Z), insbesondere zur Verwendung bei der prophylaktischen und/oder therapeutischen Behandlung von neurogenen Blasenfunktionsstörungen, insbesondere neurogenen Blasenentleerungsstörungen, vorzugsweise von mit einer Detrusorhyperaktivität und/oder einer Detrusor-Sphinkter-Dyssynergie einhergehenden neurogenen Blasenfunktionsstörungen, befüllt ist und
wobei die in der Kolbenspritze enthaltene oxybutyninhaltige Zusammensetzung (Z) einschließlich der Kolbenspritze (1) dampfsterilisiert, insbesondere wasserdampfsterilisiert, wird.

14. Dampfsterilisation, insbesondere Wasserdampfsterilisation, zur Herstellung einer vorzugsweise anwendungsfertigen, sterilen, mit einer oxybutyninhaltigen Zusammensetzung (Z) befüllten Kolbenspritze (1) wie in einem der vorangehenden Ansprüche definiert,
wobei die mit der oxybutyninhaltigen Zusammensetzung (Z) befüllte Kolbenspritze (1) und/oder die in der Kolbenspritze (1) bereitgestellte oxybutyninhaltige Zusammensetzung (Z) dampfsterilisiert, insbesondere wasserdampfsterilisiert, wird.

## Claims

1. Piston syringe (1), in particular a disposable piston syringe,
wherein the piston syringe (1) has
- a syringe body (2), which has a syringe barrel (3) for receiving a preferably oxybutynin-containing composition (Z), and an adapter (4) for connection to an instillation device, wherein the syringe body (2) is formed in one piece and/or wherein the adapter (4) is firmly and/or non-detachably connected to the syringe barrel (3), and
- a syringe plunger (5) with a plunger stopper (6);
wherein the adapter (4) has at least one first section (A₁) with an outer circumference and/or outer diameter tapering continuously in the outlet direction (AR), and
wherein the adapter (4) has at least one second, cylindrical section (A₂) directly adjoining the first section (A₁) in the outlet direction (AR) with a reduced outer circumference compared to the first section (A₁),
wherein the second section (A₂) extends towards an outlet opening (4a) of the adapter (4) and the outlet opening (4a) is formed at the free end of the second section (A₂),
wherein the first section (A₁) tapers from a maximum outside diameter (Dₘₐₓ₁) of the first section (A₁) in the outlet direction (AR) to a minimum outside diameter (Dₘᵢₙ₁) of the first section (A₁) and the ratio of the maximum outside diameter (Dₘₐₓ₁) of the first section (A₁) to the minimum outside diameter (Dₘᵢₙ₁) of the first section (A₁) is in a range from 1,1 to 1.6,
**characterized in that** exactly one step (offset, stage) (7) is formed at the transition from the first section (A₁) to the second section (A₂),
wherein at least one third section (A₃) of the adapter (4) is provided directly upstream of the first section (A₁),
wherein the third section (A₃) has an outer circumference and/or outer diameter that tapers continuously in the outlet direction (AR), wherein the third section (A₃) merges smoothly into the first section (A₁) and
wherein the ratio of a maximum outer diameter (Dₘₐₓ₃) of the third section (A₃) to a minimum outer diameter (Dₘᵢₙ₃) of the third section (A₃) is in a range from 1.01 to 1.06.

2. Piston syringe (1) according to claim 1,
wherein the adapter (4) has a cavity, in particular a passage channel (9), located between the outlet opening (4a) of the adapter (4) and the inlet opening (4b) of the adapter (4), in particular wherein the cavity, in particular the passage channel (9), extends from the inlet opening (4b) of the adapter (4) to the outlet opening (4a) of the adapter (4) and/or connects the inlet opening (4b) to the outlet opening (4a).

3. Piston syringe (1) according to claim 1 or 2,
wherein a rounded transition (8) is provided on the step (7), in particular on a region of the step (7) adjoining the second section (A₂),
in particular wherein the transition (8) has a radius in the range from 0.1 mm to 0.6 mm, preferably in the range from 0.2 mm to 0.5 mm, in particular in the range from 0.3 mm to 0.4 mm.

4. Piston syringe (1) according to one of the preceding claims,
wherein the first section (A₁) is conical; and/or
wherein the ratio of the maximum outer diameter (Dₘₐₓ₁) of the first portion (A₁) to the minimum outer diameter (Dₘᵢₙ₁) of the first portion (A₁) is in a range from 1.15 to 1.5, preferably in a range from 1.2 to 1.45, preferably in a range from 1.25 to 1.4, particularly preferably in a range from 1.3 to 1.35; and/or
wherein the ratio of the minimum outside diameter (Dₘᵢₙ₁) of the first section (A₁) to the outside diameter (D₂) of the second section (A₂) is in a range from 1.1 to 2, in particular in a range from 1.2 to 1.9, preferably in a range from 1.3 to 1.8, preferably in a range from 1.4 to 1.7, in particular in a range from 1.5 to 1.6.

5. Piston syringe (1) according to one of the preceding claims,
wherein the second section (A₂) extends from the minimum outer diameter (Dₘᵢₙ₁) of the first section (A₁) to the outlet opening (4a) of the adapter (4).

6. Piston syringe (1) according to one of the preceding claims,
wherein the third section (A₃) and the first section (A₁) have the same outer diameter at their common transition; and/or
wherein the third section (A₃) is formed and/or arranged directly adjacent to the syringe body (2), preferably to the syringe barrel (3).

7. Piston syringe (1) according to claim 6,
wherein the ratio of the maximum outside diameter (Dₘₐₓ₃) of the third section (A₃) to the minimum outside diameter (Dₘᵢₙ₃) of the third section (A₃) is in a range from 1.02 to 1.05, preferably in a range from 1.03 to 1.045, preferably in a range from 1.035 to 1.04, and/or
wherein the ratio of the maximum outer diameter (Dₘₐₓ₃) of the third section (A₃) to the minimum outer diameter (Dₘᵢₙ₁) of the first section (A₁) and/or the diameter at the step of the adapter (4) is in a range from 1.05 to 1.75, in particular in a range from 1.15 to 1.65, preferably in a range from 1.25 to 1.55, preferably in a range from 1.35 to 1.45; and/or
wherein the ratio of a maximum outer diameter (Dₘₐₓ₃) of the third section (A₃) of the adapter (4) to the outer diameter (D₂) of the second section (A₂) of the adapter (4) is in a range from 1.7 to 2.5, in particular in a range from 1.8 to 2.4, preferably in a range from 1.9 to 2.3, preferably in a range from 2 to 2.2.

8. Piston syringe (1) according to one of the preceding claims,
wherein the ratio of the length (l₁) of the first section (A₁) of the adapter (4) to the length (l₂) of the second section (A₂) of the adapter (4) is in a range from 1.1 to 1.8, in particular in a range from 1.2 to 1.7, preferably in a range from 1.3 to 1.6, preferably in a range from 1.4 to 1.5; and/or
wherein the ratio of the length (l₁) of the first section (A₁) of the adapter (4) to the length (l₃) of the third section (A₃) of the adapter (4) is in a range from 1.6 to 2.4, in particular in a range from 1.7 to 2.3, preferably in a range from 1.8 to 2.2, preferably in a range from 1.9 to 2.1; and/or
wherein the ratio of the length (l₂) of the second section (A₂) of the adapter (4) to the length (l₃) of the third section (A₃) of the adapter (4) is in a range from 1.1 to 1.8, in particular in a range from 1.2 to 1.7, preferably in a range from 1.3 to 1.6, preferably in a range from 1.4 to 1.5.

9. Piston syringe (1) according to one of the preceding claims,
wherein the syringe barrel (2) is formed in one piece by means of bonding, welding, fusing and/or casting; and/or
wherein the connection, in particular the fixed and/or non-detachable connection, preferably the material-locking connection, of the adapter (4) to the syringe barrel (3) is produced by means of bonding, welding, fusing and/or casting; and/or
wherein the adapter (4) is connected to the syringe barrel (3) by means of bonding, welding, fusing and/or casting, in particular firmly and/or non-detachably connected, preferably materially bonded; and/or
wherein the syringe barrel (2) is formed in one piece by means of injection molding, casting molding, extrusion and/or glass molding, in particular by means of injection molding, casting molding and/or extrusion; and/or
wherein the connection, in particular the fixed and/or non-detachable connection, preferably the material-locking connection, of the adapter (4) to the syringe barrel (3) is produced by means of injection molding, casting molding, extrusion and/or glass molding, in particular by means of injection molding, casting molding and/or extrusion; and/or
wherein the adapter (4) is connected to the syringe barrel (3) by means of injection molding, casting molding, extrusion and/or glass molding, in particular by means of injection molding, casting molding and/or extrusion, in particular firmly and/or non-detachably connected, preferably materially bonded; and/or
wherein the adapter (4) is at least substantially rotationally symmetrical.

10. Piston syringe (1) according to one of the preceding claims,
wherein the syringe body (2), preferably the syringe barrel (3) and the adapter (4), is or are formed from a plastic material or glass material, preferably plastic material; and/or
wherein the syringe barrel (2), preferably the syringe barrel (3) and the adapter (4), is formed from polypropylene or comprises a polypropylene; or
wherein the syringe body (2), preferably the syringe barrel (3) and the adapter (4), is or are formed from a cycloolefin (co-)polymer (COP or COC).

11. Piston syringe (1) according to one of the preceding claims,
wherein the oxybutynin-containing composition (Z) contained in the plunger syringe (1), including the plunger syringe (1) and the closure element (13), if present, is or are steam-sterilized, in particular steam-sterilized, and/or is or are present,
in particular wherein the steam sterilization is carried out *under overkill conditions* and/or at about 121 °C and/or for a period of at least 15 min, in particular of at least 20 min, preferably of about 20 min; and/or
in particular wherein the steam sterilization is carried out according to Pharmacopoea Europaea (Ph. Eur.), Chapter 5.1.1 (07/2017:50101), in particular under *overkill conditions* and/or at about 121 °C and/or for a period of at least 15 min, in particular of at least 20 min, preferably of about 20 min, and/or
in particular wherein the sterile plunger syringe (1) filled with the sterile oxybutynin-containing composition (Z) obtained after steam sterilization and/or the oxybutynin-containing composition (Z) obtained after steam sterilization has a SAL value (*Sterility Assurance Level*) of at most 10⁻⁵, in particular at most 10⁻⁶, preferably at most 10⁻⁷.

12. Kit, in particular application and/or instillation system, comprising
(i) at least one piston syringe (1) as defined in one of claims 1 to 11;
(ii) (a) at least one application and/or instillation device which can be connected to the piston syringe (1), in particular to the adapter (4) of the piston syringe (1), in particular in the form of an instillation tube or the like, preferably in the form of a (urinary bladder) catheter;
optionally (iii) an adapter counterpart for connecting the application and/or instillation device to the piston syringe (1), in particular to the adapter (4) of the piston syringe (1); and
optionally (iv) at least one application and/or instillation instruction.

13. Use of a plunger syringe (1) defined according to any one of the preceding claims in a process for the preparation of a sterile oxybutynin-containing composition (Z),
wherein the plunger syringe (1) is filled with the sterile oxybutynin-containing composition (Z), in particular for use in the prophylactic and/or therapeutic treatment of neurogenic bladder dysfunctions, in particular neurogenic voiding dysfunctions, preferably neurogenic bladder dysfunctions associated with detrusor hyperactivity and/or detrusorsphincter dyssynergia, and
wherein the oxybutynin-containing composition (Z) contained in the piston syringe including the piston syringe (1) is steam-sterilized, in particular steam-sterilized.

14. Steam sterilization, in particular water steam sterilization, for producing a preferably ready-to-use, sterile plunger syringe filled with an oxybutynin-containing composition (Z) as defined in one of the preceding claims,
wherein the piston syringe (1) filled with the oxybutynin-containing composition (Z) and/or the oxybutynin-containing composition (Z) provided in the piston syringe (1) is steam-sterilized, in particular steam-sterilized.

## Revendications

1. Seringue à piston (1), en particulier seringue à piston à usage unique,
la seringue à piston (1) ayant
- un corps de seringue (2) qui présente un cylindre de seringue (3) pour recevoir une composition (Z) contenant de préférence de l'oxybutynine, et un adaptateur (4) pour le raccordement à un dispositif d'instillation, le corps de seringue (2) étant réalisé d'une seule pièce et/ou l'adaptateur (4) étant relié au cylindre de seringue (3) de manière fixe et/ou inamovible, et
- un piston de seringue (5) avec un bouchon de piston (6)
l'adaptateur (4) présentant au moins une première section (A₁) avec une circonférence extérieure et/ou un diamètre extérieur se rétrécissant de manière continue dans la direction de sortie (AR), et
l'adaptateur (4) présentant au moins une deuxième section cylindrique (A₂) se raccordant directement à la première section (A₁) dans le sens de la sortie (AR), avec une circonférence extérieure réduite par rapport à la première section (A₁),
où la seconde partie (A₂) s'étend vers une ouverture de sortie (4a) de l'adaptateur (4) et l'ouverture de sortie (4a) est formée à l'extrémité libre de la seconde partie (A₂),
la première section (A₁) se rétrécissant dans le sens de la sortie (AR) à partir d'un diamètre extérieur maximal (Dₘₐₓ₁) de la première section (A₁) jusqu'à un diamètre extérieur minimal (Dₘᵢₙ₁) de la première section (A₁), et le rapport entre le diamètre extérieur maximal (Dₘₐₓ₁) de la première section (A₁) et le diamètre extérieur minimal (Dₘᵢₙ₁) de la première section (A₁) étant compris dans une plage de 1,1 à 1,6,
**caractérisé en ce qu'**exactement une marche (décalage) (7) est formée à la transition entre la première section (A₁) et la deuxième section (A₂),
au moins une troisième section (A₃) de l'adaptateur (4) étant prévue directement en amont de la première section (A₁),
la troisième section (A₃) présentant une circonférence extérieure et/ou un diamètre extérieur se rétrécissant de manière continue dans le sens de la sortie (AR), la troisième section (A₃) se raccordant sans palier à la première section (A₁) et
où le rapport d'un diamètre extérieur maximal (Dₘₐₓ₃) de la troisième partie (A₃) à un diamètre extérieur minimal (Dₘᵢₙ₃) de la troisième partie (A₃) est dans une plage de 1,01 à 1,06.

2. Seringue à piston (1) selon la revendication 1,
où l'adaptateur (4) présente un espace creux, en particulier un canal de passage (9), situé entre l'ouverture de sortie (4a) de l'adaptateur (4) et l'ouverture d'entrée (4b) de l'adaptateur (4), en particulier où l'espace creux, en particulier le canal de passage (9), s'étend de l'ouverture d'entrée (4b) de l'adaptateur (4) à l'ouverture de sortie (4a) de l'adaptateur (4) et/ou relie l'ouverture d'entrée (4b) à l'ouverture de sortie (4a).

3. Seringue à piston (1) selon la revendication 1 ou 2,
une transition arrondie (8) étant prévue sur la marche (7), en particulier sur une zone de la marche (7) adjacente à la deuxième section (A₂),
en particulier où la transition (8) présente un rayon dans la plage de 0,1 mm à 0,6 mm, de préférence dans la plage de 0,2 mm à 0,5 mm, en particulier dans la plage de 0,3 mm à 0,4 mm.

4. Seringue à piston (1) selon l'une quelconque des revendications précédentes,
où la première partie (A₁) est de forme conique; et/ou
où le rapport du diamètre extérieur maximal (Dₘₐₓ₁) de la première section (A₁) au diamètre extérieur minimal (Dₘᵢₙ₁) de la première section (A₁) est dans une plage de 1,15 à 1,5, de préférence dans une plage de 1,2 à 1,45, de préférence dans une plage de 1,25 à 1,4, de manière particulièrement préférée dans une plage de 1,3 à 1,35; et/ou
où le rapport entre le diamètre extérieur minimal (Dₘᵢₙ₁) de la première section (A₁) et le diamètre extérieur (D₂) de la seconde section (A₂) se situe dans une plage de 1,1 à 2, en particulier dans une plage de 1,2 à 1,9, de préférence dans une plage de 1,3 à 1,8, de préférence dans une plage de 1,4 à 1,7, en particulier dans une plage de 1,5 à 1,6.

5. Seringue à piston (1) selon l'une des revendications précédentes,
où la deuxième section (A₂) s'étend à partir du diamètre extérieur minimal (Dₘᵢₙ₁) de la première section (A₁) vers l'ouverture de sortie (4a) de l'adaptateur (4).

6. Seringue à piston (1) selon l'une des revendications précédentes,
où la troisième section (A₃) et la première section (A₁) ont le même diamètre extérieur à leur transition commune; et/ou
où la troisième section (A₃) est formée et/ou disposée de manière directement adjacente au corps de seringue (2), de préférence au cylindre de seringue (3).

7. Seringue à piston (1) selon la revendication 6,
où le rapport du diamètre extérieur maximal (Dₘₐₓ₃) de la troisième section (A₃) sur le diamètre extérieur minimal (Dₘᵢₙ₃) de la troisième section (A₃) est dans une plage de 1,02 à 1,05, de préférence dans une plage de 1,03 à 1,045, de préférence dans une plage de 1,035 à 1,04, et/ou
où le rapport du diamètre extérieur maximal (Dₘₐₓ₃) de la troisième section (A₃) au diamètre extérieur minimal (Dₘᵢₙ₁) de la première section (A₁) et/ou du diamètre au niveau de l'étage de l'adaptateur (4) est dans une plage de 1,05 à 1,75, en particulier dans une plage de 1,15 à 1,65, de préférence dans une plage de 1,25 à 1,55, de préférence dans une plage de 1,35 à 1,45; et/ou
où le rapport d'un diamètre extérieur maximal (Dₘₐₓ₃) de la troisième section (A₃) de l'adaptateur (4) sur le diamètre extérieur (D₂) de la deuxième section (A₂) de l'adaptateur (4) est dans une plage de 1,7 à 2,5, en particulier dans une plage de 1,8 à 2,4, de préférence dans une plage de 1,9 à 2,3, de préférence dans une plage de 2 à 2,2.

8. Seringue à piston (1) selon l'une des revendications précédentes,
où le rapport entre la longueur (l₁) de la première partie (A₁) de l'adaptateur (4) et la longueur (l₂) de la seconde partie (A₂) de l'adaptateur (4) est compris dans une plage de 1,1 à 1,8, en particulier dans une plage de 1,2 à 1,7, de préférence dans une plage de 1,3 à 1,6, de préférence dans une plage de 1,4 à 1,5; et/ou
où le rapport entre la longueur (l₁) de la première partie (A₁) de l'adaptateur (4) et la longueur (l₃) de la troisième partie (A₃) de l'adaptateur (4) est compris dans une plage de 1,6 à 2,4, en particulier dans une plage de 1,7 à 2,3, de préférence dans une plage de 1,8 à 2,2, de préférence dans une plage de 1,9 à 2,1; et/ou
où le rapport entre la longueur (l₂) de la deuxième section (A₂) de l'adaptateur (4) et la longueur (l₃) de la troisième section (A₃) de l'adaptateur (4) est compris dans une plage de 1,1 à 1,8, en particulier dans une plage de 1,2 à 1,7, de préférence dans une plage de 1,3 à 1,6, de préférence dans une plage de 1,4 à 1,5.

9. Seringue à piston (1) selon l'une des revendications précédentes,
le corps de la seringue (2) étant formé d'une seule pièce par collage, soudage, fusion et/ou coulée; et/ou
la liaison, en particulier la liaison fixe et/ou indétachable, de préférence la liaison par la matière, de l'adaptateur (4) avec le cylindre de seringue (3) étant produite par collage, soudage, fusion et/ou coulée; et/ou
l'adaptateur (4) étant relié au cylindre de seringue (3) par collage, soudage, fusion et/ou coulée, en particulier relié de manière fixe et/ou indissociable, de préférence relié par liaison de matière; et/ou
le corps de seringue (2) étant formé d'une seule pièce au moyen d'un moulage par injection, d'un moulage par coulée, d'une extrusion et/ou d'un moulage en verre, en particulier au moyen d'un moulage par injection, d'un moulage par coulée et/ou d'une extrusion; et/ou
la liaison, en particulier la liaison solide et/ou indétachable, de préférence la liaison par matière, de l'adaptateur (4) avec le cylindre de seringue (3) étant produite par moulage par injection, moulage par coulée, extrusion et/ou moulage de verre, en particulier par moulage par injection, moulage par coulée et/ou extrusion; et/ou
l'adaptateur (4) étant relié au cylindre de seringue (3) par moulage par injection, moulage par coulée, extrusion et/ou moulage de verre, en particulier par moulage par injection, moulage par coulée et/ou extrusion, en particulier relié de manière fixe et/ou indétachable, de préférence relié par liaison de matière; et/ou
l'adaptateur (4) étant conçu au moins sensiblement à symétrie de révolution.

10. Seringue à piston (1) selon l'une des revendications précédentes,
le corps de seringue (2), de préférence le cylindre de seringue (3) et l'adaptateur (4), étant formé d'une matière plastique ou d'une matière vitreuse, de préférence d'une matière plastique; et/ou
le corps de seringue (2), de préférence le cylindre de seringue (3) et l'adaptateur (4), étant formé de polypropylène ou comprend un polypropylène; ou
le corps de seringue (2), de préférence le cylindre de seringue (3) et l'adaptateur (4), étant formé d'un (co)polymère de cyclooléfine (COP ou COC).

11. Seringue à piston (1) selon l'une des revendications précédentes,
où la composition (Z) contient de l'oxybutynine contenue dans la seringue à piston (1), y compris la seringue à piston (1) et l'élément de fermeture (13) éventuellement présent, est ou sont stérilisés à la vapeur, en particulier stérilisés à la vapeur d'eau, et/ou est ou sont présents,
en particulier où la stérilisation à la vapeur est effectuée dans *des conditions de surchauffe* et/ou à environ 121 °C et/ou pendant une période d'au moins 15 minutes, en particulier d'au moins 20 minutes, de préférence d'environ 20 minutes; et/ou
en particulier où la stérilisation à la vapeur est effectuée conformément à la Pharmacopoea Europaea (Ph. Eur.), chapitre 5.1.1 (07/2017:50101), notamment dans *des conditions de surfusion* et/ou à environ 121 °C et/ou pendant une durée d'au moins 15 minutes, en particulier d'au moins 20 minutes, de préférence d'environ 20 minutes, et/ou
en particulier où la seringue à piston stérile (1) remplie de la composition stérile contenant de l'oxybutynine (Z) obtenue après stérilisation à la vapeur et/ou la composition contenant de l'oxybutynine (Z) obtenue après stérilisation à la vapeur présente une valeur SAL (*Sterility Assurance Level*) de 10⁻⁵ au maximum, en particulier de 10⁻⁶ au maximum, de préférence de 10⁻⁷ au maximum.

12. Kit, notamment système d'application et/ou d'instillation, comprenant (i) au moins une seringue à piston (1) telle que définie dans l'une quelconque des revendications 1 à 11; (ii) (a) au moins un dispositif d'application et/ou d'instillation pouvant être raccordé à la seringue à piston (1), notamment à l'adaptateur (4) de la seringue à piston (1), notamment sous la forme d'un tuyau d'instillation ou analogue, de préférence sous la forme d'un cathéter (urinaire); le cas échéant (iii) un adaptateur-contrepartie pour raccorder le dispositif d'application et/ou d'instillation à la seringue à piston (1), en particulier à l'adaptateur (4) de la seringue à piston (1); et le cas échéant (iv) au moins une notice d'application et/ou d'instillation.

13. Utilisation d'une seringue à piston (1) définie selon l'une quelconque des revendications précédentes dans un procédé de préparation d'une composition stérile (Z) contenant de l'oxybutynine,
la seringue à piston (1) étant remplie de la composition stérile contenant de l'oxybutynine (Z), en particulier pour une utilisation dans le traitement prophylactique et/ou thérapeutique de troubles neurogènes de la fonction vésicale, en particulier de troubles neurogènes de la miction, de préférence de troubles neurogènes de la fonction vésicale associés à une hyperactivité du détrusor et/ou à une dyssynergie détrusor-sphincter, et
la composition contenant de l'oxybutynine (Z) contenue dans la seringue à piston étant stérilisée à la vapeur, en particulier stérilisée à la vapeur d'eau, y compris la seringue à piston (1).

14. Stérilisation à la vapeur, en particulier à la vapeur d'eau, pour la fabrication d'une seringue à piston stérile, de préférence prête à l'emploi, remplie d'une composition (Z) contenant de l'oxybutynine, telle que définie dans l'une des revendications précédentes,
où la seringue à piston (1) remplie de la composition contenant de l'oxybutynine (Z) et/ou la composition contenant de l'oxybutynine (Z) préparée dans la seringue à piston (1) est stérilisée à la vapeur, en particulier stérilisée à la vapeur d'eau.
